# EUROPEAN PATENT APPLICATION

(11) **EP 2 894 476 A1**
(43) Date of publication of application: **15.07.2015**
(21) Application number: 14151074.3
(22) Date of filing: 14.01.2014
(51) Int. Cl.: G01N 33/58, C07K 1/00, G01N 33/68

(54) **Genetically Encoded Spin Label**

(71) Applicant: Universität Konstanz, 78464 Konstanz (DE)
(72) Inventor: Schmidt, Moritz Johannes, 78464 Konstanz (DE); Drescher, Malte, 78467 Konstanz (DE); Summerer, Daniel, 78462 Konstanz (DE)
(74) Representative: Elbel, Michaela

(57) **Abstract**

The invention relates to a non-canonical amino acid of the formula I, namely A - L - X, A is a lysine or a tyrosine, L is a linker or absent, and X is an aminoxyl radical, and if A is lysine, L is bound to the N-epsilon atom of the lysine or, if L is absent, X is bound to the N-epsilon atom of the lysine; and if A is tyrosine, L is bound to the phenolic hydroxyl of the tyrosine or, if L is absent, X is bound to the phenolic hydroxyl of the tyrosine. Moreover, the invention also relates to a method for introducing a spin label into a protein and to a modified pyrrolysyl-tRNA-synthetase.

## Description

### Field of the Invention

The invention relates to a non-canonical amino acid of the formula I, namely A - L - X, wherein A is a lysine or a tyrosine, L is a linker or absent, and X is an aminoxyl radical. The invention further relates to a method for introducing a spin label into a protein and to a method for analyzing a protein comprising a non-canonical amino acid of the invention. Moreover, the invention relates to a modified pyrrolysyl-tRNA-synthetase and to the use of a non-canonical amino acid for site-directed spin labeling.

### Background of the Invention

Electron paramagnetic resonance (EPR) spectroscopy in combination with site-directed spin labeling (SDSL) is a powerful tool to study the structure, dynamics and interactions of proteins (Jeschke 2012). In particular, it is suitable to reveal detailed information about the binding interface of proteins on the structural level and to investigate kinetic and thermodynamic aspects of protein interactions. EPR is based on the excitation of electron spins of unpaired electrons within a molecule. However, most molecules, as e.g. proteins, do not comprise an unpaired electron by nature, such that chemical groups having an unpaired electron need to be introduced into a protein for EPR spectroscopy analysis. This is usually achieved by site-directed spin labeling, wherein a spin label comprising a free electron is introduced into the protein to be investigated at a specific position. For example, a cysteine residue is artificially introduced into the protein, e.g. by site-directed mutagenesis, and subsequently reacted with a spin label e.g. methanethiosulfonate spin label (MTSSL) (Altenbach et al., 1990). This way of introducing a spin label has, however, significant disadvantages. First of all, the reaction is mediated by the generation of a disulfide bond between the cysteine and the MTSSL, such that an introduction using other amino acids is not possible. Moreover, the protein to investigate must not comprise any further cysteine residue, as the reaction with MTSSL would otherwise be unspecific. Therefore, in most cases endogenous cysteine residues of the protein need to be replaced by amino acid substitution. Additionally, cysteine residues are particularly reactive due to their SH-group and therefore, often contribute to the specific folding of the protein or even to its enzymatically active centers. Thus, replacing all cysteine groups of a protein will most likely alter its structure and/or functionality, reducing the informative value of the EPR analysis. Additionally, the disulfide bonds formed between the cysteine and the MTSSL are rather sensitive to reducing agents, which significantly limits the chemical agents, which can be used during the analysis procedures. Finally, the described approach may only be used in extracellular environments, since the cysteine cannot be chemically reacted within a cell.

As an alternative approach, spin labels may be introduced into proteins by chemically synthesizing the entire protein using Fmoc or Boc based step by step peptide synthesis (Klare et al., 2009). This was used, for example, to introduce TOAC (2,2,6,6-tetramethylpiperidine-1-oxyl-4-amino-4-carboxyclic acid) into proteins. Chemically synthesized proteins, however, often deviate at least slightly from their natural equivalents, since during cellular protein translation folding is usually supported by specific helper proteins. Moreover, chemical synthesis of proteins is limited to molecules of about 150 amino acids, and is likewise restricted to extracellular approaches.

Therefore, there is a need in the art to provide tools and methods to completely obviate the need for chemical labeling steps in protein EPR distant measurements.

### Summary of the Invention

In a first aspect, the invention relates to a non-canonical amino acid (ncAA) of the formula I, namely A - L - X, wherein A is a lysine or a tyrosine, L is a linker or absent, and X is an aminoxyl radical, wherein if A is lysine, L is bound to the N-epsilon atom of the lysine or, if L is absent, X is bound to the N-epsilon atom of the lysine; and if A is tyrosine, L is bound to the phenolic hydroxyl of the tyrosine or, if L is absent, X is bound to the phenolic hydroxyl of the tyrosine.

In a further aspect, the invention relates to a ncAA selected from the group consisting of 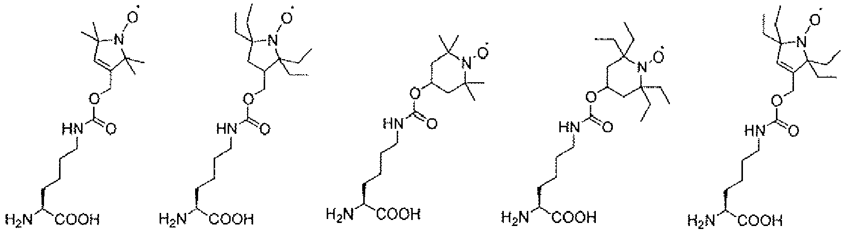 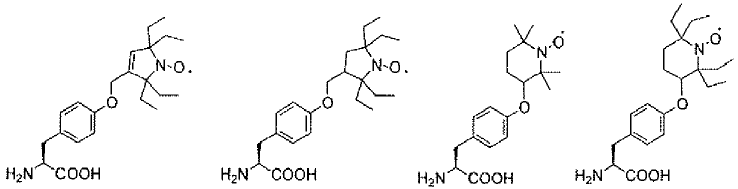 and

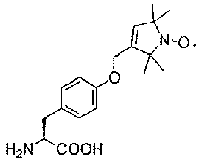

In a further aspect, the invention relates to a protein comprising at least one, preferably two ncAA of the invention.

In a further aspect, the invention relates to a method for introducing a spin label into a protein comprising the steps of providing a nucleic acid sequence encoding for the protein, introducing a stop codon or a quadruplet codon into the sequence, and expressing the sequence in the presence of a tRNA^{Pyl}, a modified PylRS, and a non-canonical amino acid (ncAA) of the invention, which is encoded by the stop codon or the quadruplet codon.

In a further aspect, the invention relates to a method for analyzing a protein comprising the steps of producing a derivative of the protein comprising at least one non-canonical amino acid (ncAA) of the invention at a defined position, and recording the electron paramagnetic resonance spectrum of the derivative.

In a further aspect, the invention relates to a modified pyrrolysyl-tRNA-synthetase (PylRS) having a substitution of alanine for tyrosine at position 306, a substitution of phenylalanine for tyrosine at position 384 and a substitution of leucine for isoleucine at position 413.

In a further aspect, the invention relates to a modified pyrrolysyl-tRNA-synthetase (PylRS) having a substitution of alanine for tyrosine at position 306, a substitution of phenylalanine for tyrosine at position 384 and a substitution of threonine, alanine, valine or lysine for asparagine at position 346.

In a further aspect, the invention relates to the use of a non-canonical amino acid (ncAA) according to the invention for site-directed spin labeling.

### Brief Description of the Drawings

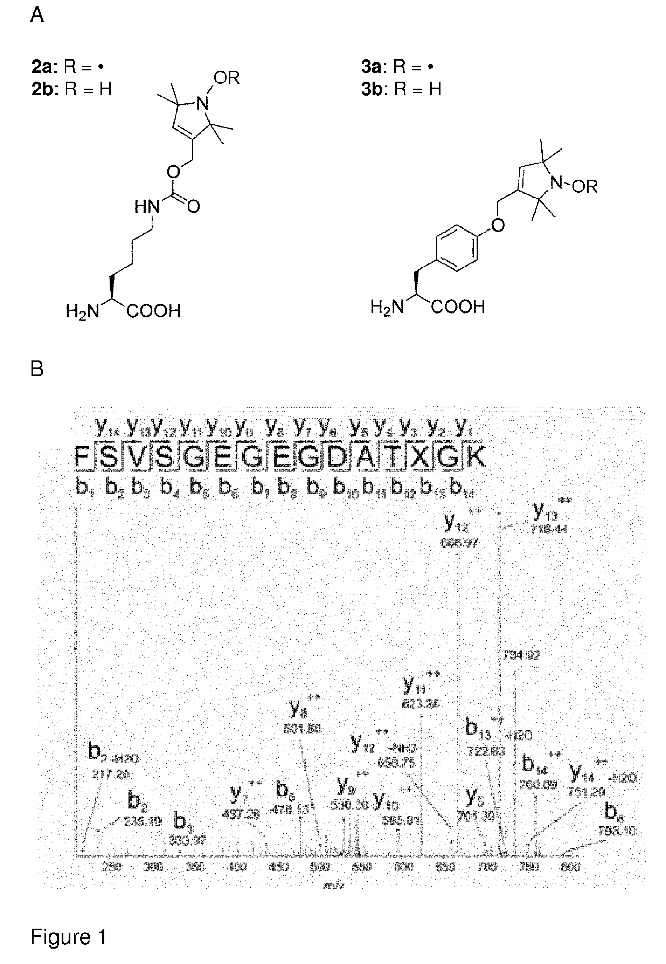

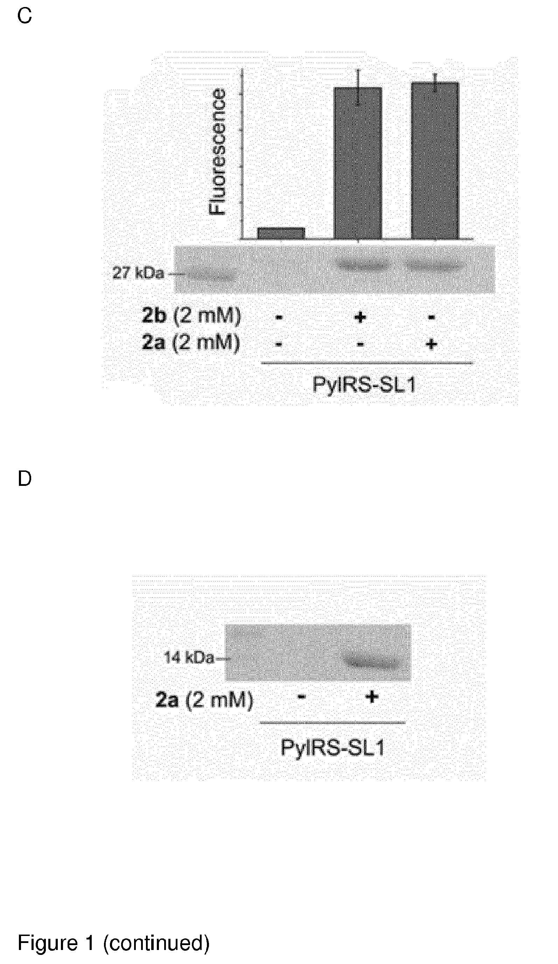

Figure 1 shows structures of a lysine derivative comprising a 2,2,5,5-tetramethylpyrroline-1-oxyl residue (**2a**) and the hydroxylamine variant thereof (**2b**), as well as a tyrosine derivative comprising a 2,2,5,5-tetramethylpyrroline-1-oxyl residue (**3a**) and the hydroxylamine variant thereof (**3b**) (A), ESI MS/MS spectrum of DTT-reduced GFP-Y39→2a tryptic peptide fragment (**2a** incorporation site is marked as X) (**B**), the expression of GFP-Y39→**2a** and GFP-Y39→**2b** with C-terminal His6 tag under co-expression of tRNA^{Pyl}/PylRS-SL1 (Top: Cellular GFP fluorescence, Bottom: SDS PAGE analysis of proteins purified by Ni-NTA chromatography) (C), and a SDS PAGE analysis of purified TRX-R74→**2a** expressions under co-expression of tRNA^{Pyl}/PylRS-SL1 (D).

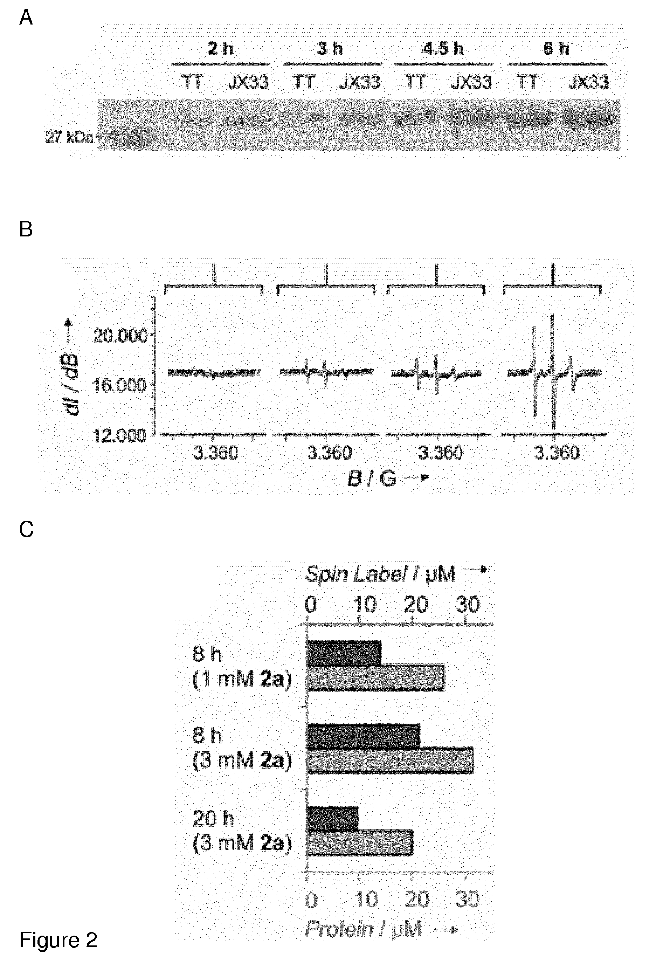

Figure 2 shows the direct biosynthesis of spin-labeled proteins in *E*. *coli.* Expression kinetic of GFP-Y39→**2a** in *E. coli* strains Top10 (TT) and JX33 analyzed by SDS PAGE (A), EPR measurements of GFP-Y39→**2a** samples from Figure 2A (from Top10) (B), and quantification of the labeling degree of GFP-Y39→**2a**. Concentration of GFP protein was quantified with a BCA assay (light grey bars) and that of **2a** by EPR measurements (C). Labeling degree was obtained as the ratio of the concentration of **2a** and the concentration of GFP in percent.

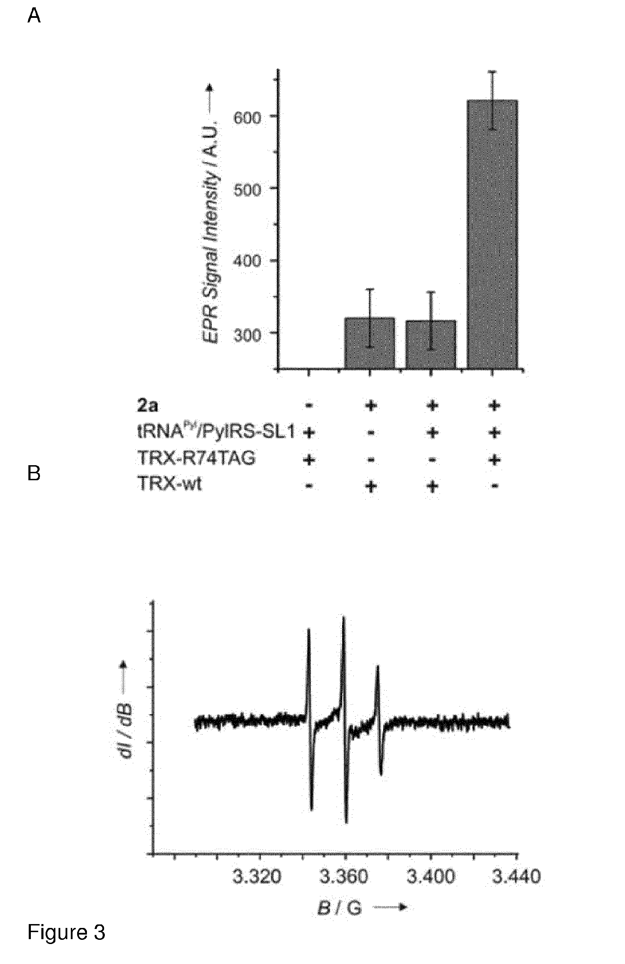

Figure 3 shows in-cell EPR measurement in *E*. *coli.* EPR signal intensities for in-cell EPR measurements of washed *E*. coli cells expressing TRX-wt or TRX-R74TAG and not expressing or co-expressing tRNA^{Pyl}/PylRS-SL1 in presence or absence of 2a as A.U. = arbitrary units (A). EPR spectrum of washed *E*. *coli* cells expressing *E*. *coli* TRX-R74→**2a** at 4°C (B).

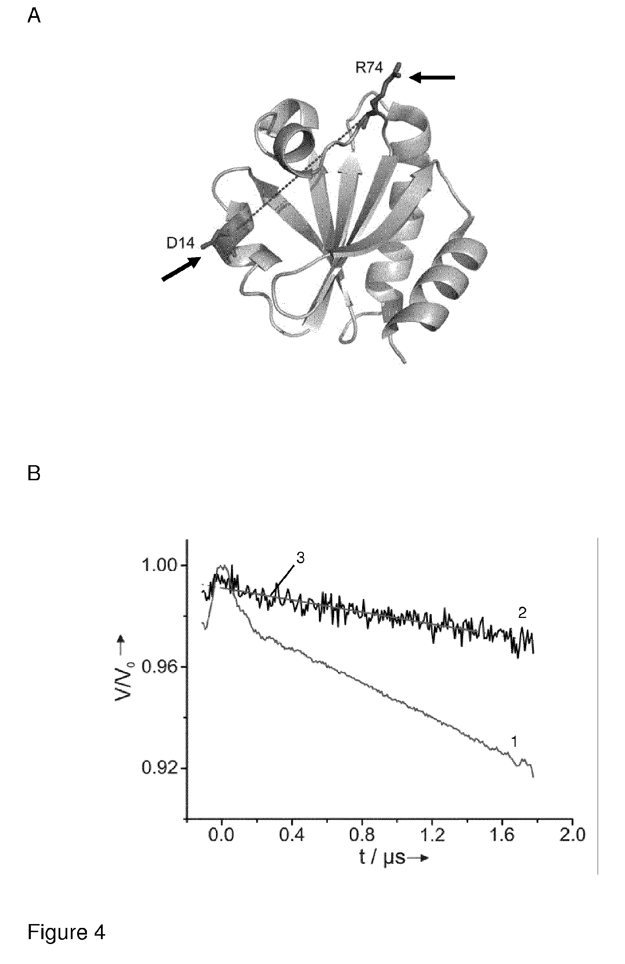

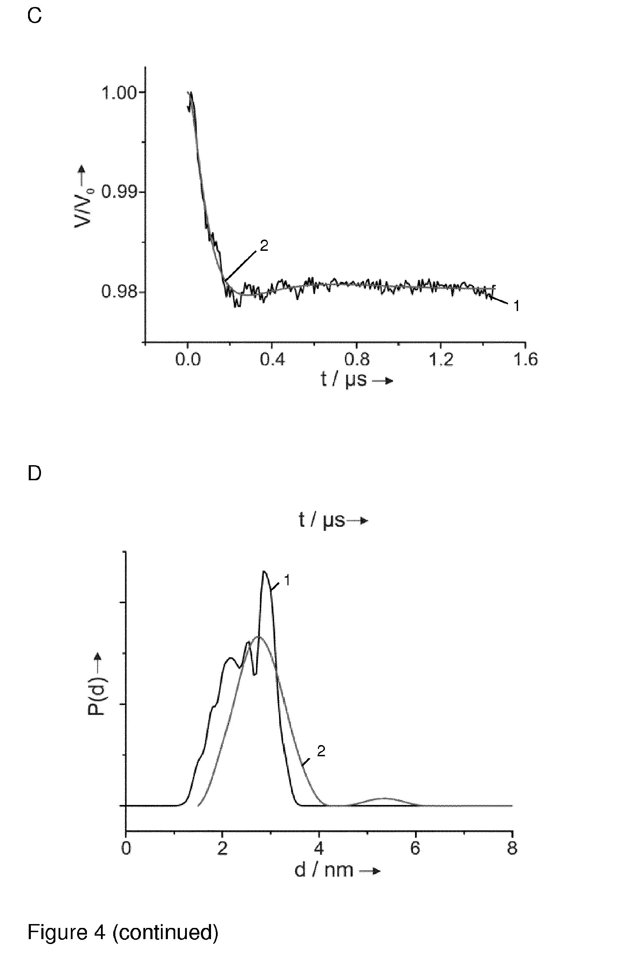

Figure 4 shows EPR distance measurements between two amino acids **2a** incorporated into *E. coli* thioredoxin (TRX). Cartoon representation of a crystal structure of TRX (pdb entry 2TRX). Amino acids at both incorporation sites of **2a** indicated by arrows, the corresponding C_{α} -C_{α} distance is indicated as dotted line (A). DEER raw data of doubly labelled TRX-D14/R74→**2a** (lower graph, marked 1) and TRX-D14→**2a** as control (upper, noisy graph, marked 2. Obtained graph with fit of data for TRX-D14→**2a** is marked with 3 (B). DEER data of TRX-D14/R74→**2a** upon background correction (marked 1) and fit based on a model free analysis (marked 2) (C). Distance distribution for TRX-D14/R74→**2a** corresponding to the fit shown in C (marked 1) compared to the theoretically predicted distance distribution between conventional MTSSL labels (marked 2) (D).

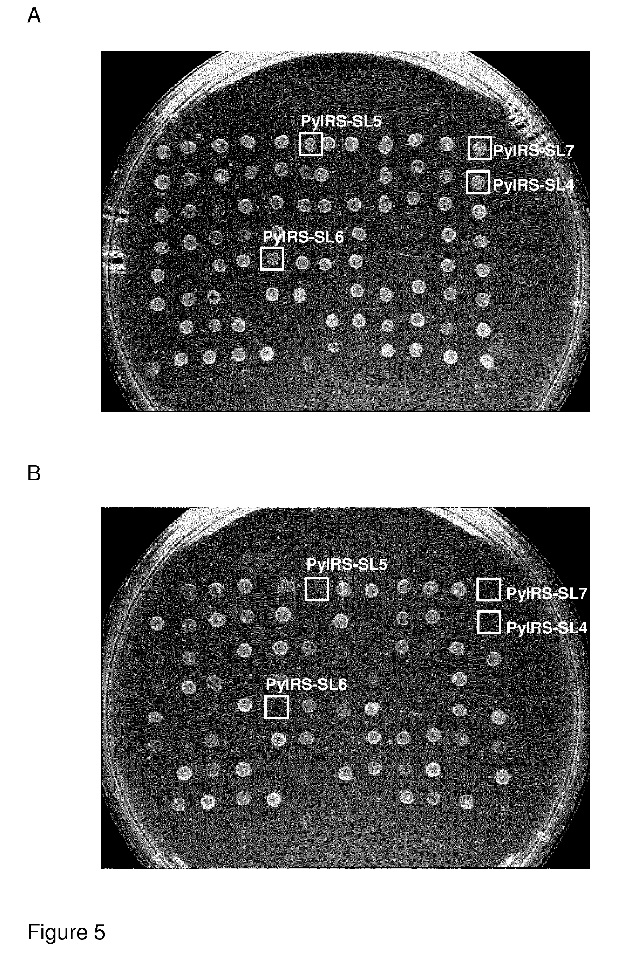

Figure 5 shows a growth assay with chloramphenicol for identifying PylRS mutants, which are able to process tyrosine derivatives comprising a 2,2,5,5-tetramethyl-pyrroline-1-oxyl residue. Cells expressing individual modified PylRS and an ambermutant of chloramphcnicol-acetyl-transferase (CAT98TAG) were grown in the presence (A) and the absence (B) of a ncAA. The assay identified the PylRS mutants PyIRS-SL4, PylRS-SL5, PylRS-SL6, and PylRS-SL7.

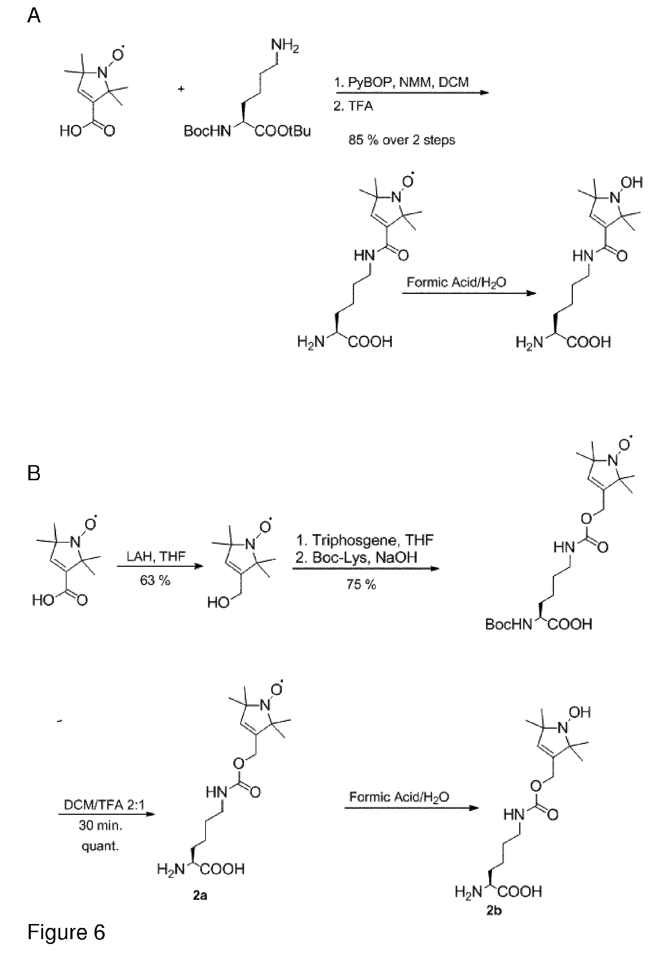

Figure 6 shows an overview of the synthesis of (S)-2-amino-6-(1-oxy-2,2,5,5-tetramethy(pyrroline-3-carboxamido}hexanoic acid and (S)-2-amina-6-(1-hydroxy-2,2,5,5-tetramethylpyrroline-3-carboxamido)hexanoic acid (A), and of (S)-2-amino-6-((((1-oxy-2,2,5,5-tetramethylpyrroline-3-yl)methoxy)carbonyl)amino)hexanoic acid **2a** and (S)-2-amino-6-((((1-hydroxy-2,2,5,5-tetramethylpyrroline-3-yl)methoxy)carbonyl)-amino)hexanoic acid **2b** (B).

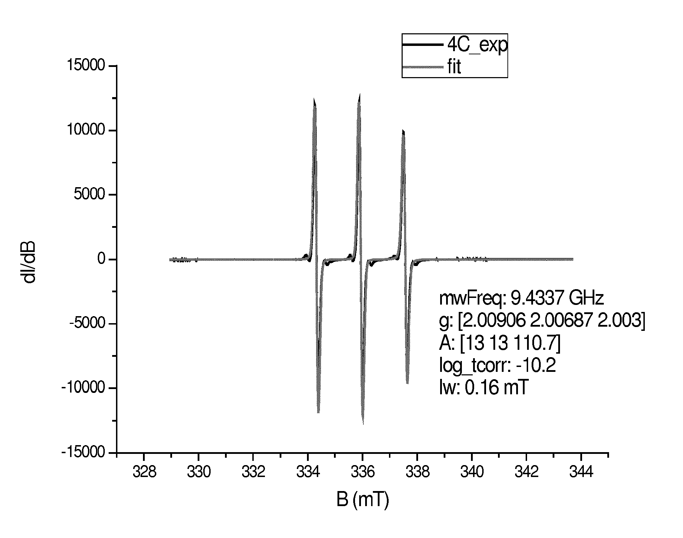

Figure 7 shows the EPR spectrum of ncAA **2a** with full characterization.

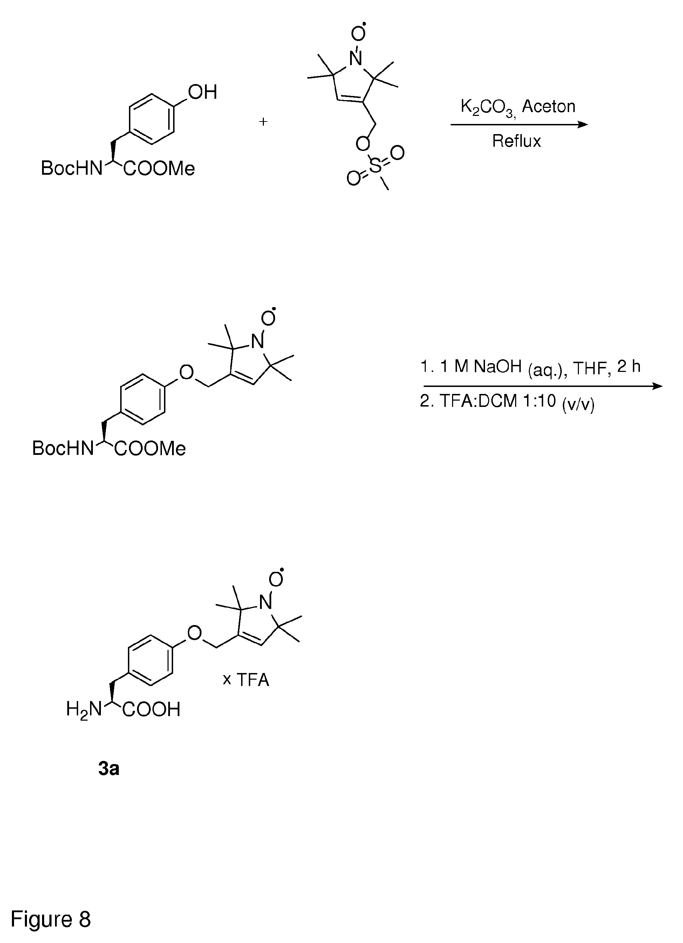

Figure 8 shows an overview of the synthesis of a tyrosine derivative comprising a 2,2,5,5-tetramethylpyrroline-1-oxyl residue (**3a**).

### Detailed Description of the Invention

In a first aspect, the invention relates a non-canonical amino acid (ncAA) of the formula I

A-L-X

wherein A is a lysine or a tyrosine,
L is a linker or absent, and
X is an aminoxyl radical,
wherein if A is lysine, L is bound to the N-epsilon atom of the lysine or, if L is absent, X is bound to the N-epsilon atom of the lysine;
and if A is tyrosine, L is bound to the phenolic hydroxyl of the tyrosine or, if L is absent, X is bound to the phenolic hydroxyl of the tyrosine.

The ncAA of the invention combines two different properties. First, it is a derivative of a natural amino acid, namely lysine or tyrosine, such that it can form an integral part of a peptide or protein without disturbing the molecule's structure. Second, it comprises an aminoxyl radical, which comprises an unpaired electron and is therefore suitable for EPR spectroscopy. Thus, the ncAA of the invention can be used for producing proteins for EPR distance measurements without the need of chemical labeling steps.

Since the ncAA has the basic structure of lysine or tyrosine, it can be introduced into a peptide or protein by common methods of protein synthesis. The term "protein" as used herein refers to a compound produced by amide formation between a carboxyl group of one amino acid and an amino group of another amino acid. The term comprises amino acid polymers of any length including oligopeptides, polypeptides as well as large proteins.

The ncAA may be incorporated into a protein by cellular or cell-free protein synthesis by the use of an orthogonal tRNA/aminoacyl-tRNA-synthetase (aaRS) pair. For example, the ncAA may be incorporated into a peptide by using *E. coli* cells expressing a mutant pyrrolysyl-tRNA-synthetase, which harbors a Y384F and a Y306A mutation (PylRS_AF) (Yanagisawa et al., 2008). Due to said mutations, the PylRS_AF has a ncAA binding pocket, which is considerably enlarged compared to that of wild type PylRS, and can accommodate a ncAA comprising an aminoxyl radical. Using such a modified PylRS, functional proteins comprising a 2,2,5,5-tetramethyl-pyroline-1-oxyl residue were obtained (Figure 2).

By incorporating the ncAA into a peptide, the resulting molecule comprises the aminoxyl radical of the ncAA. The term "aminoxyl radical" as used herein refers to compounds comprising the structure R₂N-O⁻. Such compounds are radicals derived from hydroxylamines by removal of the hydrogen atom from the hydroxy group. Aminoxyl radicals may also be referred to as "nitroxyl radicals" or "nitroxides" (International Union of Pure and Applied Chemistry, IUPAC). Due to the free electron comprised in the aminoxyl radical, the residue is suitable for EPR spectroscopy. This technique is based on measuring the magnetic moment of free electrons, namely the electron spin. By exposing a probe comprising a free electron to a continuous or pulsed electromagnetically alternating field, a resonance absorption occurs, which is determined by the environment of the free electron. Thereby, information on the structure and the dynamics of the molecule at the position of the free electron can be obtained. By integrating the free electron in form of an aminoxyl radical into a natural amino acid, the free electron may be introduced into any protein at any given position.

This allows analyzing proteins by EPR spectroscopy independently of their individual sequence. In particular, it is not necessary to alter amino acids other than the one comprising the aminoxyl radical. Moreover, since the ncAA is a derivative of a natural amino acid and aminoxyl radicals are rather small residues, the ncAA may be introduced at most positions without interfering with the protein's structure or function. Additionally, the incorporation of the ncAA may be achieved within a cell, i.e. by native protein synthesis mechanisms. This results in proteins, which resemble their native equivalent most closely, in particular with respect to correct folding. Finally, introducing the ncAA by cell-based systems or cell-free assays, is less elaborate and costly compared to chemical protein synthesis.

In a preferred embodiment, L is selected from the group consisting of an ether residue, a carbamate residue and an urea residue. In general, the linker between the amino acid and the aminoxyl radical should be rather small to allow an efficient interaction with the PylRS, resulting in a more efficient incorporation of the ncAA into a protein. Ether bonds, carbamate bonds and urea bonds were found to be particularly suitable as linkers between the amino acid and the aminoxyl radical. Moreover, they allow an easy and straight-forward chemical synthesis of the ncAA.

In a preferred embodiment, if A is lysine, L is a carbamate residue and/or if A is tyrosine, L is an ether residue. Such ncAAs were found to be efficiently incorporated into proteins, in particular using PylRS_AF, PylRS-SL1, PylRS-SL2 or PylRS-SL3 when processing a lysine derivative and using PylRS-SL4, PylRS-SL5, PylRS-SL6 or PylRS-SL7 when processing a tyrosine derivative, respectively.

In a preferred embodiment, the aminoxyl radical comprises an optionally substituted heterocycle, preferably an optionally substituted pyrroline residue, an optionally substituted oxazolidine residue or an optionally substituted piperidine residue.

In a further preferred embodiment, the aminoxyl radical comprises a 2,2,5,5-tetramethylpyrroline-1-oxyl residue, a 2,2,5,5-tetraethylpyrroline-1-oxyl residue, a 2,2,6,6-tetramethylpiperidine-1-oxyl residue, a 2,2,6,6-tetraethylpiperidine-1-oxyl residue, a 2,2,5,5-tetramethylpyrrolidine-1-oxyl residue, a 2,2,5,5-tetraethylpyrrolidine-1-oxyl residue or a 4,4-dimethyl-oxazolidine-*N*- oxyl residue.

Accordingly, in a particularly preferred embodiment, the aminoxyl radical is selected from the group consisting of

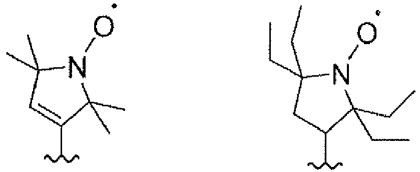

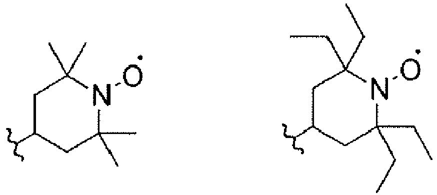

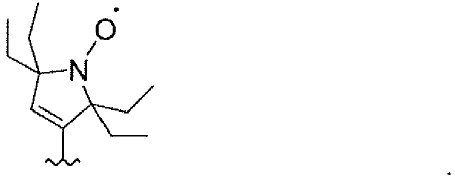

These aminoxyl radicals were found to be particular suitable for EPR spectroscopy. More specifically, 2,2,5,5-tetramethylpyrroline-1-oxyl residues exhibited an exceptionally high stability in *E*. *coli.* NcAAs comprising this aminoxyl radical resulted in very high yields of spin labeled proteins. Importantly, the observed protein levels were sufficient to allow intracellular measurements, such that the protein could be analyzed in its natural environment. Thus, using the ncAA of the invention the protein of interest can be synthesized, labeled, processed and investigated within the same environment, which even may be the native environment of the protein, i.e. a cell or cell type, in which it naturally occurs.

In a further aspect, the invention relates to a non-canonical amino acid (ncAA) selected from the group consisting of 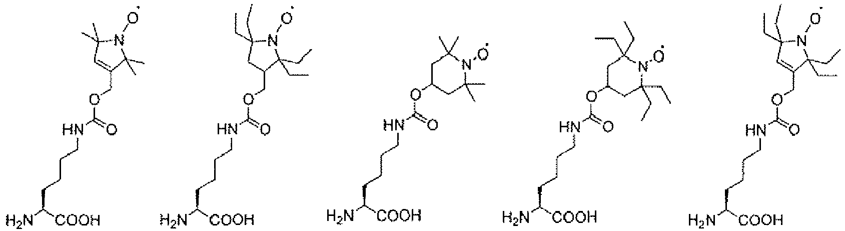 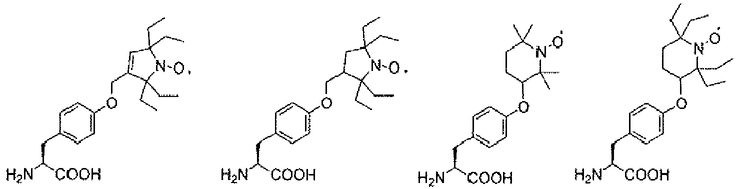 and

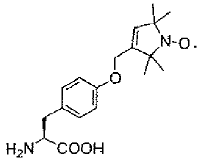

Lysine and tyrosine derivatives comprising a 2,2,5,5-tetramethyl-pyrroline-1-oxyl-moiety were efficiently incorporated into proteins using the modified PylRS described herein providing sufficient yields of spin labeled proteins for EPR spectroscopy.

In a further aspect, the invention relates to a protein comprising at least one, preferably two ncAAs of the invention. By introducing the ncAA of the invention into a protein, the structure of the protein directly surrounding the free electron of the aminoxyl radical can be investigated by EPR spectroscopy. Moreover, two ncAAs may be introduced into the protein at different positions, such that double electron-electron resonance (DEER, also referred to as Pulsed Electron Double Resonance, PELDOR,) spectroscopy can be performed. These measurements rely on the dipole-dipole coupling between the spin labels, which is inversely proportional to the cube of their distance and are therefore precise over a broad range of distances (McHaourab et al., 1996). Pulsed methods can be used to separate this dipole-dipole interaction from other contributions of the spin Hamiltonian (Schweiger and Jeschke, 2005). This specific type provides information on interactions within a molecule that would not be detectable using common EPR spectra. For example, DEER spectroscopy allows analysis of distances and the orientation of two free electrons in the range of nanometres providing highly precise and reliable information on the investigated molecule. Likewise, the two ncAAs may be incorporated in two different but interacting proteins and DEER may used to examine the interaction between these proteins.

In a further aspect, the invention is directed to a method for introducing a spin label into a protein comprising the steps of providing a nucleic acid sequence encoding for the protein, introducing a stop codon or a quadruplet codon into the sequence, and expressing the sequence in the presence of a tRNA^{Pyl}, a modified PylRS and a non-canonical amino acid of the invention, which is encoded by the stop codon or the quadruplet codon. By binding the aminoxyl radical to the lysine or tyrosine, a residue comprising a free electron can be integrated into any protein by common peptide bonds by the process of translation. Therefore, spin labeled proteins can be produced without the need of chemical modification, which reduces the risk of disturbing the proteins' structure by introducing the spin label. The introduction of the ncAA into the protein can be achieved either by using a cellular or a cell-free translation system (also called *in vitro* translation assay). The term "cellular translation system" as used herein refers to a cell or organism, which is modified such that it is suitable to express the protein of the invention when supplemented with a ncAA and a respective orthogonal tRNA/aaRS pair. The cell provides all molecules necessary for protein translation, in particular endogenous tRNAs, natural amino acids and ribosomes. Cells for protein production may be microorganisms, such as prokaryotic cells, preferably bacterial cells, or eukaryotic cells, preferably cells of a fungus. Cellular translation systems are commonly used for the production of recombinant proteins, with *Escherichia coli, Saccharomyces* and *Bacillus* most widely applied. The term "cell-free translation system" as used herein refers to *in vitro* systems comprising all compounds necessary for functional protein translation. The systems are commonly based on cell extracts, e.g. from *E. coli,* wheat germ or rabbit reticulocytes, and contain ribosomes, translation factors and tRNAs. Alternatively, they can be reconstituted from individually expressed and purified components, as in so-called PURE-systems. Depending on the application, the system may be supplemented with further compounds, e.g. RNA polymerases.

Using either translation system, a nucleic acid molecule encoding the protein sequence serves as a template (mRNA). For incorporating the ncAA of the invention into the protein encoded by the mRNA, a unique codon is introduced into the mRNA at the position corresponding to the position of the ncAA in the protein. This unique codon is assigned to the ncAA through the use of an orthogonal aaRS/tRNA pair. Thus, the genetic code is expanded by a further codon, which is translated by the protein translation machinery into the ncAA (Liu and Schultz, 2010). For ensuring specificity, the codon assigned to the ncAA is unique, preferably a stop codon or quadruplet codon. In the course of translation, the ribosome produces an amino acid chain according to the nucleic acid sequence of the mRNA incorporating the ncAA.

When using cellular translation systems, the cells may be modified to express a tRNA responding to the unique codon and an aminoxyl-tRNA-synthetase for transferring the ncAA to the tRNA. For example, the tRNA may be tRNA^{Pyl}, which responds to an amber codon and the ncAA may be transferred thereon by a modified pyrrolysine-tRNA-synthetase as described herein. For cell-free translation systems, the assay may be supplemented with ncAA already loaded onto a tRNA.

In a preferred embodiment, the sequence is expressed in a cellular translation system or an *in vitro* translation assay. When using a cellular translation system, such as a microorganism, it is sufficient to provide the cells with nucleic acid sequences encoding the tRNA, the aminoacyl-tRNA-synthetase and the mRNA of the protein, and supplement the medium of the cells with the ncAA. The ncAA is transported into the cell cytoplasm where it is transferred onto the tRNA by the aminoacyl-tRNA-synthetase. Moreover, most microorganisms may be cultured in large quantities, thereby providing sufficient yields of protein for EPR analysis. Additionally, expressing the protein in a cellular translation system also allows analyzing the labeled protein within the cell, namely in a natural environment. Since EPR spectroscopy is particularly suitable for analyzing protein-protein interactions using the ncAA and the protein of the invention, these interactions can be observed *in vivo.*

In cell-free translation systems, in contrast, all components need to be added as functional molecules or their subunits. However, the translation process can be controlled more stringently in such systems and isolation of the produced peptides is easier.

In a preferred embodiment, the modified PylRS comprises a substitution of alanine for tyrosine at position 306 (Y306A) and a substitution of phenylalanine for tyrosine at position 384 (Y384F). The term "modified PylRS" as used herein refers to a pyrrolysine-tRNA-synthetase, which differs from the wild type PylRS (SEQ ID NO.: 1) by amino acid exchanges that result in an enlargement of the ncAA binding pocket such that the binding pocket, can accommodate an aminoxyl radical residue. A suitable modified PylRS is for example PylRS_AF harboring a Y384F and a Y306A mutation (PylRS_AF, Yanagisawa et al., 2008). In addition to this,the inventors found three further PylRS mutants suitable for transferring aminoxyl comprising lysine derivatives: PylRS-SL1 (SEQ ID NO.: 2), PyIRS-SL2 (SEQ ID NO.: 3) and PyIRS-SL3 (SEQ ID NO.: 4). All of these derivatives comprise a substitution of alanine for tyrosine at position 306, a substitution of phenylalanine for tyrosine at position 384 and a substitution of leucine for isoleucine at position 413. Two of the modified PylRS, PyIRS-SL2 and PyIRS-SL3, comprise a further modification, namely a substitution of arginine for lysine at position 415 and a substitution of threonine for cysteine at position 348, respectively. A Summary of the amino acid substitutions of the mutant PylRS is given in Table 2. Accordingly, in a further preferred embodiment, the modified PylRS comprises a substitution of alanine for tyrosine at position 306, a substitution of phenylalanine for tyrosine at position 384 and a substitution of leucine for isoleucine at position 413. These modified PylRS are particularly suited for processing lysine derivatives comprising an aminoxyl residue.

In an alternatively preferred embodiment, the modified PylRS comprises a substitution of alanine for tyrosine at position 306, a substitution of phenylalanine for tyrosine at position 384 and a substitution of threonine, alanine, valine or lysine for asparagine at position 346. Surprisingly, although all aminoacyl-tRNA-synthetases are specific for a single amino acid, thereby contributing to the reliability of the cellular translation mechanism, the inventors identified modified PylRSs, which are suitable to process tyrosine derivatives comprising an aminoxyl radical, PylRS-SL4 (SEQ ID NO.: 5), PylRS-SL5 (SEQ ID NO.: 6), PyIRS-SL6 (SEQ ID NO.: 7) and PyIRS-SL7 (SEQ ID NO.:8). Interestingly, in addition to the Y306A and Y384F mutations, all of these modified PylRS comprise a substitution for replacing the asparagine at position 346, indicating that the replacement of this amino acid mediates the adaption of the PylRS for processing tyrosine derivatives, at least partially. However, the substituting amino acid turned out to be less specific, as the asparagine could be replaced by any of threonine, alanine, valine or lysine. A Summary of the amino acid substitutions of the mutant PylRS is given in Table 2.

In a further aspect, the invention relates to a method for analyzing a protein comprising the steps of producing a derivative of the protein comprising at least one non-canonical amino acid (ncAA) of the invention at a defined position, and recording the electron paramagnetic resonance spectrum of the derivative. By introducing the ncAA, into a protein, this protein can be analyzed using EPR. Depending on the position of the ncAA within the protein, specific parts of the protein, namely the immediate environment of the free electron of the aminoxyl radical, can be analyzed. The ncAA may be integrated into the protein at any desired position, because, as a derivative of a natural amino acid, it is less likely to interfere with the protein's structure or function.

In a preferred embodiment, the derivative comprises two non-canonical amino acids (ncAA) of the invention at two distinct positions. Since the aminoxyl radical is introduced into the protein by use of a derivative of a natural amino acid, it can be introduced at any desired position with minimized risk of interfering with the protein's structure or function. Moreover, the aminoxyl residue is introduced as an integral part of the amino acid derivative such that it is not necessary to chemically react the entire protein as e.g. for MTSSL. This further allows introducing not only one but two free electrons into one protein or into two proteins forming a homomeric or heteromeric complex. A protein or protein complex comprising two ncAAs can be analyzed using DEER spectroscopy. This particular kind of EPR spectroscopy is suitable for determining the distance and orientation of two free electrons. Thereby information is obtained on the protein's or protein complex' architecture and conformational changes within the protein or protein complex, providing significant insight into the molecular properties of proteins.

In a preferred embodiment, the method further comprises the step of producing a derivative of a second protein comprising an ncAA of the invention at a defined position. By introducing two ncAAs into two distinct molecules, the interaction of the two molecules can be analyzed using DEER for obtaining information e.g. about the amino acids contributing to the protein-protein interactions, the binding surfaces and the kinetics and thermodynamics of the interaction. The method of the invention may by applied on homomeric protein-complexes by providing two distinct derivatives of the same protein or on heteromeric protein-complexes by incorporated ncAAs into different proteins.

In a preferred embodiment, producing the derivative comprises providing a nucleic acid sequence encoding for the protein, introducing a stop codon or a quadruplet codon into the sequence, and expressing the sequence in the presence of a tRNA^{Pyl}, a modified PylRS, and the non-canonical amino acid, which is encoded by the stop codon or the quadruplet codon. Moreover, the derivative of the protein may be expressed in an *in vitro* expression system.

In a further preferred embodiment, the modified PylRS has a substitution of alanine for tyrosine at position 306, a substitution of phenylalanine for tyrosine at position 384 and a substitution of leucine for isoleucine at position 413 and, optionally a further substitution of threonine for cysteine at position 348 and/or of arginine for lysine at position 415. These modified PylRS are particularly suited for processing lysine derivatives comprising an aminoxyl radical.

In an alternatively preferred embodiment, the modified PylRS has a substitution of alanine for tyrosine at position 306, a substitution of phenylalanine for tyrosine at position 384 and a substitution of threonine, alanine, valine or lysine for asparagine at position 346, and optionally a further substitution of tryptophan, lysine or tyrosine for cysteine at position 348, or of valine for isoleucine at position 413. The modified PylRS may further comprise a substitution of phenylalanine or alanine at position 302 and of valine for leucine at position 309. These modified PylRS are particularly suited for processing tyrosine derivatives comprising an aminoxyl radical.

In a further aspect, the invention relates to a modified pyrrolysyl-tRNA-synthetase (PylRS) having a substitution of alanine for tyrosine at position 306, a substitution of phenylalanine for tyrosine at position 384 and a substitution of leucine for isoleucine at position 413. The inventors identified three different modified PylRS molecules (PylRS-SL1 (SEQ ID NO.: 2), PylRS-SL2 (SEQ ID NO.: 3) and PylRS-SL3 (SEQ ID NO.: 4)), which were suitable to process lysine derivatives comprising an aminoxyl radical. The identified modified PylRS all showed a substitution of leucine for isoleucine at position 413, indicating that this modification mediates the ability of the modified PylRS to accommodate aminoxyl radical comprising amino acids, at least partially.

In a particularly preferred embodiment, the modified PylRS may further comprise a substitution of threonine for cysteine at position 348 and/or a substitution of arginine for lysine at position 415. Each of PylRS-SL1, PylRS-SL2 and PylRS-SL3 are suitable to generate tRNAs comprising a lysine derivative of the invention, e.g. lysine derivatives of the following:

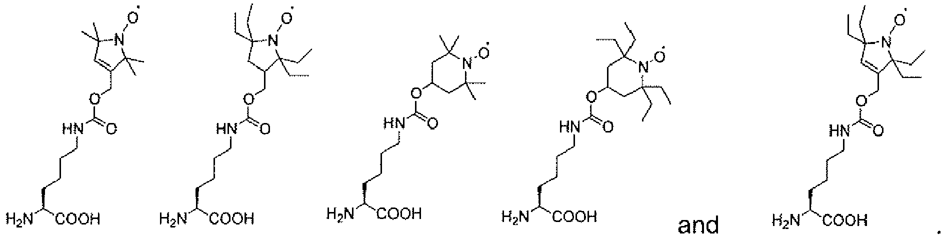

In a further aspect, the invention relates to a modified pyrrolysyl-tRNA-synthetase (PylRS) having a substitution of alanine for tyrosine at position 306, a substitution of phenylalanine for tyrosine at position 384 and a substitution of threonine, alanine, valine or lysine for asparagine at position 346. Additionally to the above-mentioned modified PylRS, the inventors identified four PylRS mutants, which are able to process derivatives of tyrosine PylRS-SL4 (SEQ ID NO.: 5), PylRS-SL5 (SEQ ID NO.: 6), PylRS-SL6 (SEQ ID NO: 7) and PylRS-SL7 (SEQ ID NO.: 8). Interestingly, all of these mutants comprise a substitution at position 346 replacing the asparagine located at that position. However, the identity of the substituting amino acid turned out to be less crucial, as the asparagine could be substituted by threonine, alanine, valine or even lysine. All four mutants are suited for efficiency generating tRNAs comprising tyrosine derivatives, e.g. tyrosine derivative of the following

In a preferred embodiment, the modified pyrrolysyl-tRNA-synthetase (PylRS) has at least one additional mutation at position 348 and/or 413. PyIRS-SL4, PyIRS-SL5 and PyIRS-SL6 show an additional substitution at position 348, which occurs, however, to be less specific, since the cysteine located at position 348 may be substituted for tryptophan, lysine or tyrosine. PylRS-SL7 was found to have an additional substitution of valine for isoleucine at position 413. 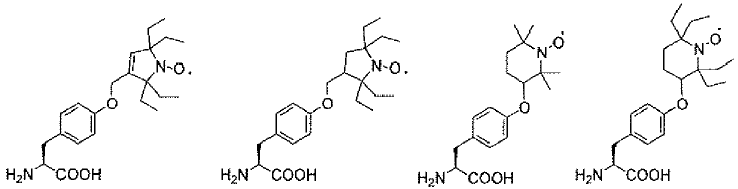 and

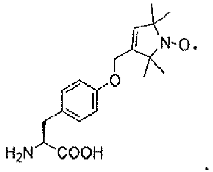

In a further aspect, the invention relates to a modified PylRS selected from the group consisting of PylRS-SL1, PylRS-SL2, PylRS-SL3, PylRS-SL4, PylRS-SL5, PylRS-SL6 and PylRS-SL7.

In a further aspect, the invention relates to a plasmid encoding a modified PylRS of the invention. In particular, the PylRS may be encoded by DNA sequences as follows:

**Table 1: PylRS protein and DNA sequences, respectively:**

| PylRS | Protein sequence | DNA sequence |
|---|---|---|
| PylRS-SL1 | SEQ ID NO.: 2 | SEQ ID NO.: 9 |
| PylRS-SL2 | SEQ ID NO.: 3 | SEQ ID NO.: 10 |
| PylRS-SL3 | SEQ ID NO.: 4 | SEQ ID NO.: 11 |
| PylRS-SL4 | SEQ ID NO.: 5 | SEQ ID NO.: 12 |
| PylRS-SL5 | SEQ ID NO.: 6 | SEQ 10 NO.: 13 |
| PylRS-SL6 | SEQ ID NO.: 7 | SEQ ID NO.: 14 |
| PylRS-SL7 | SEQ ID NO.: 8 | SEQ ID NO.: 15 |

In a further aspect, the invention relates to the use of a modified PylRS of the invention for producing a protein with a non-canonical amino acid of the invention.

In a further aspect, the invention relates to a non-canonical amino acid of the invention for site-directed spin labeling.

In a further aspect, the invention relates to a ncAA of the invention for in-cell EPR spectroscopy. Recently, first proof-of-principle experiments for in-cell EPR measurements have been reported. However, these studies required technical demanding microinjection of heterologous, *in vitro* spin labeled biomolecules in high concentrations into *Xenopus laevis* oocytes as model cells. Using the ncAA of the invention, the spin labeled protein can be produced within a cell and, thus, examined in its natural environment.

### Examples

### Material and Methods

### Synthesis of ncAAs

All reactions were performed under anhydrous conditions and an inert atmosphere of argon in oven-dried glassware with magnetic stirring. Yields refer to chromatographically and spectroscopically (¹H NMR) homogenous materials. Reagents were used as obtained from typical commercial sources. Flash chromatography was carried out using Roth Kieselgel 60 F254 (230-400 mesh) silica gel. Solvents used for chromatography were technical grade. Petrol ether had a range of boiling points of 35 - 80 °C. ¹H and ¹³C NMR spectra were recorded at ambient temperature on a Bruker Avance III 400 instrument. Signal positions were reported in δ/ppm with their characteristics denoted as s (singlet), d (doublet), dd (double doublet), t (triplet), m (multiplet) and br (broad). High-resolution mass spectrometry (HRMS) measurements were performed on a Bruker Daltonics micrOTOF II. A summary of the synthesis is depicted in Figure 6.

### Synthesis of lysine derivatives

A summary of the way of synthesis is depicted in Figure 6.

Synthesis of (S)-tert-butyl 2-((tert-butoxycarbonyl)amino)-6-(1-oxy-2,2,5,5-tetramethyl-pyrroline-3-carboxamido)hexanoate: Boc-Lys-0-tBu (410 mg, 1.36 mmol, 1 eq.) and carboxy-2,2,5,5-tetramethyl-3-pyrroline-1-yloxy, free radical (250 mg, 1.36 mmol, 1 eq) were dissolved in anhydrous DCM (25 ml) under argon atmosphere. The solution was cooled in an ice-bath and N-methylmorpholine (328 µl, 2.99 mmol, 2.2 eq) and PyBOP (benzotriazol-1-yl-oxytripyrrolidinophosphanium hexafluorophosphate (777 mg, 1.49 mmol, 1.1 eq) were added. The reaction mixture was stirred for 16 h at room temperature. The solution was diluted with DCM (30 ml), washed with saturated NaHCO₃, dried over MgSO₄ and the solvent was evaporated under reduced pressure. (S)-tert-butyl-2-((tert-butoxycarbonyl)amino)-6-(1-oxy-2,2,5,5-tetramethylpyrroline-3-carboxamido)hexanoate was obtained after flash column chromatography (petrol ether/ethyl acetate 1:1) as a yellow solid (570 mg, 1.22 mmol, 90 %).

Synthesis of (S)-2-amino-6-(1-oxy-2,2,5,5-tetramethylpyrroline-3-carboxamido)hexanoic acid: (S)-tert-butyl-2-((tert-butoxycarbonyl)amino)-6-(1-oxy-2,2,5,5-tetramethylpyrroline-3-carboxamido)hexanoate (385 mg, 0.82 mmol) was dissolved in DCM/TFA (4 ml, 1:1, v/v) and was stirred for 2 h at room temperature. The solvent was removed and the residue was co-evaporated with MeOH (2x 30 ml) and diethyl ether (3x 20 ml) to yield the TFA salt of (S)-2-amino-8-(1-oxy-2,2,5,5-tetramethylpyrroline-3-carboxamido)-hexanoic acid as a yellow solid (327 mg, 0.77 mmol, 94 %).

Synthesis of (S)-2-amino-6-(1-hydroxy-2,2,5,5-tetramethylpyrroline-3-carboxamido)-hexanoic acid: (S)-2-amino-6-(1-oxy-2,2,5,5-tetramethylpyrroline-3-carboxamido)-hexanoic acid (50 mg, 0.12 mmol) was dissolved in 4 ml H₂O/formic acid (1:1, v/v) and was stirred for 24 h at room temperature. The solvent was removed under reduced pressure and was co-evaporated three times with 50 mM HCl (15 ml) to afford the HCl salt of (S)-2-amino-6-(1-hyroxy-2,2,5,5-tetramethylpyrroline-3-carboxamido)hexanoic acid (39 mg, quant.).

Synthesis of 3-hydroxymethyl-1-oxy-2,2,5,5-tetramethylpyrroline: 3-carboxy-2,2,5,5-tetramethyl-3-pyrroline-1-yloxy, free radical (1 g, 5.43 mmol, 1 eq) was dissolved in anhydrous THF (30 ml) under argon atmosphere and cooled in an ice bath. Lithium aluminium hydride (237 mg, 6.24 mmol, 1.15 eq) was added in small portions and the solution was allowed to warm up to room temperature and was stirred for 48 h. The reaction mixture was quenched with water, filtered and the filtrate was extracted with ethyl acetate (5 x 50 ml). The organic phase was dried with MgSO₄ and the solvent was removed under reduced pressure. The residual yellow oil was purified by flash column chromatography (petrol ether/ethyl acetate 1:1) to yield 3-hydroxymethyl-1-oxy-2,2,5,5-tetramethylpyrroline as a yellow solid (578 mg, 3.4 mmol, 63 %).

Synthesis of (S)-2-((tert-butoxycarbonyl)amino)-6-((((1-oxy-2,2,5,5-tetramethylpyrroline-3-yl)methoxy)carbonyl)amino)hexanoic acid: 3-hydroxymethyl-1-oxy-2,2,5,5-tetramethylpyrroline (300 mg, 1.76 mmol, 1 eq) was dissolved in anhydrous THF (20 ml) under argon atmosphere and was cooled in an ice bath. Triphosgene (523 mg, 1.76 mmol, 1 eq) was added in one portion and the solution was allowed to warm up to room temperature. The reaction mixture was stirred over night (18 h) and was then added dropwise to an ice-cooled solution of Boc-Lys-OH (651 mg, 2.64 mmol, 1.5 eq) in 1 M NaOH (40 ml). The solution was stirred for 24 h, was acidified with HCl to pH = 1 and extracted with ethyl acetate (3 x 100 ml). The organic phase was dried with MgSO₄ and the solvent was removed under reduced pressure. The residual yellow oil was purified by flash column chromatography (DCM to DCM/MeOH 98:2) to yield (S)-2-((tert-butoxycarbonyl)amino)-6-((((1-oxy-2,2,5,5-tetramethylpyrroline-3-yl)methoxy)carbonyl)-amino)hexanoic acid as a yellow foam (624 mg 1.41 mmol, 75 %).

Synthesis of (S)-2-amino-6-((((1-oxy-2,2,5,5-tetramethylpyrroline-3-yl)methoxy)carbonyl)amino}hexanoic acid **2a**: (S)-2-((tert-butoxycarbonyl)amino)-6-((((1-oxy-2,2,5,5-tetramethylpyrroline-3-yl)methoxy)carbonyl)-amino)hexanoic acid (624 mg, 1.41 mmol, 1 eq) was dissolved in DCM/TFA (4.5 ml, 2:1 v/v) and was stirred 30 min at room temperature. The solvent was removed and the reaction mixture was co-evaporated with MeOH (2x 50 ml) and diethyl ether (3x 50 ml) to yield (S)-2-amino-6-((((1-hydroxy-2,2,5,5-tetramethyl-2,5-dihydro-1H-pyrrol-3-yl)methoxy)carbonyl)amino)hexanoic acid as an off-white foam (635 mg, 1.39 mmol, quant.).

Synthesis of (S)-2-amino-6-((((1-hydroxy-2,2,5,5-tetramethylpyrroline-3-yl)methoxy)-carbonyl)amino)hexanoic acid 2b: (S)-2-amino-6-((((1-hydroxy-2,2,5,5-tetramethyl-pyrroline-3-yl)methoxy)carbonyl)amino)hexanoic acid (100 mg, 0.22 mmol) was dissolved in 6 ml H₂O/formic acid (1:1, v/v) and was stirred for 24 h at room temperature. The solvent was removed under reduced pressure and was co-evaporated three times with 50 mM HCl (15 ml) to afford the HCl salt of (S)-2-amino-6-((((1-hydroxy-2,2,5,5-tetramethylpyrroline-3-yl)methoxy)carbonyl)amino)hexanoic acid (81 mg, quant.).

### Synthesis of tyrosine derivatives

A summary of the way of synthesis is depicted in Figure 8.

3-Hydroxymethyl-1-oxy-2,2,5,5-tetramethylpyrroline, free radical (640 mg, 3.76 mmol) is dissolved in DCM (40 ml) and triethylamine (0,625 ml, 4.5 mmol) under argon atmosphere and cooled in an ice bath. Methansulfonyl chloride (0,349 ml, 4.5 mmol) is added dropwise and the solution is stirred for 30 min at 0 °C and then at room temperature for 2 h. The organic phase is washed with water (20 ml) and sat. NaHCO₃ (20 ml), dried with MgSO₄ and evaporated. The residual oil was purified using flash column chromatography (petrol ether/ethyl acetate 1:2 v/v) to yield (1-hydroxy-2,2,5,5-tetramethyl-2,5-dihydro-1H-pyrrol-3-yl)methyl methanesulfonate (801 mg, 3.23 mmol, 86 %) as a yellow solid.

N-tert-Butoxycarbonyl-L-tyrosine methyl ester (794 mg, 2.69 mmol), (1-hydroxy-2,2,5,5-tetramethyl-2,5-dihydro-1H-pyrrol-3-yl)methyl methanesulfonate (801 mg, 3.23 mmol) and K₂CO₃ (892 mg, 6.45 mmol) were resuspended in aceton (80 ml) and heated at 65 °C for 19 h. Aceton was removed under redcued pressure and the reaction mixture was diluted with ethyl acetate and washed with water (2 x 50 ml). The organic phase was separated, dried with MgSO₄, evaporated and purified using flash column chromatography (petrol ether/ethyl acetate 3:1) to yield methyl (S)-2-((tert-butoxycarbonyl)amino)-3-(4-((1-hydroxy-2,2,5,5-tetramethyl-2,5-dihydro-1 H-pyrrol-3-yl)methoxy)phenyl)propanoate as a yellow foam (1,039 g, 2.32 mmol, 86 %).

Methyl (S)-2-((tert-butoxycarbonyl)amino)-3-(4-((1-hydroxy-2,2,5,5-tetramethyl-2,5-di-hydro-1H-pyrrol-3-yl)methoxy)phenyl)propanoate (1,039 g, 2.3 mmol) was dissolved in 10 ml THF and 1 M NaOH (8 ml) was added dropwise. The solution was stirred at r.t. for 1 h. The reaction mixture was washed 2x with 50 ml EtOAc and the residual aqueous solution was acidifyied with 1 M HCl and extracted into EtOAc (2x 50 ml). The organic phase was dried with MgSO₄ and the solvent was removed in vacuo. The residual yellow foam was dissolved in 10 ml TFA/DCM (1:10 v/v) and stirred until TLC indicated complete consumption. The residual oil was co-evaporated once with methanol and 5 times with diethyl ether. The yellow solid (829 mg, 1.85 mmol, 80%) was dried in vacuum.

### Expression and purification of GFP mutants containing non-canonical amino acids

*E. coli* Top10 or JX33 transformed with pEVOL-PyIRS plasmids and pBAD_GFP-39TAG were induced at an OD₆₀₀ of -0.8 with 0.2 % (w/v) L-arabinose in presence of non-canonical amino acid. The cultures were harvested after 2 - 16 h of incubation, lyzed with B-Per lysis reagent (Thermo Scientific) and purified over Ni-NTA agarose (Qiagen). Purity and identity of the obtained proteins was analyzed by SDS PAGE and staining with Gelcode blue (Thermo Scientific) as well as ESI MS and quantified using a BCA assay (Pierce).

### Measurement of cellular GFP fluorescence

Cell pellets from GFP expressions performed as described above were washed twice with PBS, resuspended in PBS and fluorescence was determined using a Tecan M200 plate reader using and excitation wavelength of 475 nm and an emission wavelength of 510 nm. Fluorescence values were corrected for cell densities based on OD₆₀₀ measurements of the cell suspensions.

### Expression and purification of wild type thioredoxin and mutant TRX-R74→2a

*E. coli* Top10 transformed with plasmids pEVOL-PylRS-SL1 (pMoS266) and pBAD-TRX_R74TAG (pSuE185, encoding *E. coli* thioredoxin with C-terminal S-tag and His-tag and an amber mutation at position R74) were induced at an OD₆₀₀ of 0.9 with 0.2 % (w/v) L-arabinose in presence of 2 mM **2a**. After 4 hours growth, cells were lyzed with B-Per lysis reagent (Thermo Scientific) and purified over Ni-NTA agarose (Qiagen). Purity and identity of the obtained protein was analyzed by SDS PAGE and staining with Gelcode blue (Thermo Scientific) as well as ESI MS and quantified using a BCA assay (Pierce).

### Growth assay of selected M. mazei PylRS mutants

A single clone of *E. coli* JX33 co-transformed with pREPDaS_PylRS-SL1 (pMoS269) and pBAD_GFP-39TAG was picked, grown over night in LB media containing 50 µg/ml carbenicillin and 25 µg/ml tetracyclin and a dilution was printed on LB agar containing the same antibiotics, 0.2 % (w/v) arabinose, varying concentrations of chloramphenicol and 2 mM **2a** or no **2a**. Plates were incubated at 37 °C and pictures taken with a digital camera using white light illumination from top. The other PylRS mutants were grown alike.

### CW-EPR measurement procedures

Continuous wave (cw-) EPR spectra were recorded at T = 20°C except for spectra of in-cell measurements, which were measured at T = 4 °C. CW-EPR experiments were performed on an X-band MiniScope spectrometer (MS200, magnettech GmbH) equipped with a variable temperature unit (Temperature Controller TC-H02, magnettech GmbH). Samples were loaded into glass capillaries (Bluebrand, outer diameter 1 mm) with typical sample volumes of 10 µl. Spectra were obtained with a modulation amplitude of 800 mG and microwave attenuation of 15 dB. The signal-to-noise ratio was improved by accumulation of typically 10 spectra featuring 120 s scan time each. The spectra were analyzed using Matlab R2008b (The MatWorks, Inc.) and the toolbox EasySpin 2.6. Signal intensities were determined via the double integral of the first derivative EPR spectrum.

### EPR characterization of 2a

In order to determine the g- and the hyperfine tensor for **2a**, a 5 mM aqueous solution of **2a** containing 20 % (vol./vol.) glycerol was transferred into a sample tube (Bruker, outer diameter 3 mm) and shock frozen in liquid nitrogen. The cw-EPR spectrum at T = 120 K was measured on an X-band Elexsys E580 spectrometer (Bruker Biospin) equipped with an Elexsys MS 3 probehead and helium gas flow system (ESR900, Oxford Instruments) using 1 G modulation amplitude, 25 dB microwave attenuation, and accumulations of 20 scans. The spectral analysis resulted in g = (2.00806, 2.00806, 2.003) and A = (11.25, 11.25, 97.47) MHz.

### Determination of labeling degree by EPR

The effective spin concentrations of protein samples were determined by quantitative spectral simulations of the cw-EPR spectra and calibration using a series of reference samples with known spin concentration ((1-oxyl-2,2,5,5-tetramethylpyrroline-3-methyl) methanethiosulfonate in aqueous solution, ranging from 1 µM to 2 mM). Determination of the effective spin concentration of the Ni-NTA extracts and comparison with the independently determined protein concentration by a BCA assay (Pierce) resulted in the labeling degree (see Figure 2C).

### Intracellular EPR measurements

A single clone of *E. coli* JX33 co-transformed with a.) plasmids pEVOL_PylRS-SL1 and pBAD-TRX_R74TAG, b.) plasmids pEVOL_PylRS-SL1 and pBAD-TRX_wt (pSuE177) or c.) only pBAD-TRX_R74_wt was picked and grown in LB media (supplemented with chloramphenicol and/or carbenicillin). This culture was diluted 100-fold into fresh LB media (10 ml) and was grown at 37 °C with shaking at 180 rpm. At an OD = 0.8, 3 mM of **2a** was added, the expression was induced with 0.2 % (w/v) L-arabinose and the culture was incubated for 16 h at 37 °C with shaking at 180 rpm. The culture was harvested by centrifugation (4000 rpm, 4 °C, 10 min) and was washed three times with 10 ml LB media + 20 % glycerol (v/v), transferred to an Eppendorf tube and washed with 1 ml LB + 20 % glycerol (v/v). The bacterial suspension was pelleted (4000 rpm, 4 °C, 10 min) and transferred into glass capillaries (Bluebrand, outer diameter 1 mm) for EPR measurements at T = 4 °C.

### DEER experiments

All EPR DEER experiments were performed in Q-band using an Elexsys E580 spectrometer (Bruker Biospin) equipped with a helium gas flow system (CF935, Oxford Instruments). The four-pulse, dead time free DEER sequence is given by: π/2_{obs} - τ₁ - π_{obs} - t - πₚᵤₘₚ - (τ₁+τ₂-t) - π_{obs} - τ₂ - echo. The pump pulse (28 ns corresponding to a π-pulse) was set to the maximum of the nitroxide spectrum and the observer pulse was set 50 MHz lower; π/2 and π pulses at observer frequency were of 24 and 48 ns length, respectively. All samples were measured at τ₂=2 µs and the accumulation time per sample was 13.6 hours at T= 50 K.

### DEER data analysis

Processing and distance distribution analysis of all DEER time traces were performed using the DeerAnalysis2013 software (Jeschke et al., 2006). An experimental background function was derived from the individually measured DEER traces of TRX-D14→**2a**. The distance distribution was extracted with the model-free Tikhonov regularization method (Chiang et al., 2005). The modulation depth of the DEER curve is significantly reduced by reduction but still sufficient to be able to obtain meaningful distances from a fit (Jeschke et al., 2000). In order to systematically analyze the influence of the different steps in the signal treatment process (baseline correction, signal to noise ratio, choice of regularization parameter etc.) on the results of Tikhonov regularization, systematic variation of parameters and monitoring changes in the resulting distance distribution has been conducted using DeerAnalysis2013. The analysis clearly indicated that the Tikhonov regularization remains stable for different regularization parameters. Distances above 4.5 nm are artefacts due to Tikhonov regularization, suppression of those distances has been shown not to affect the r.m.s.d. Since the uncertainties in the parameters discussed above may be correlated, a statistical analysis of the distance distribution obtained by variations in a multidimensional parameter space has been conducted (1000 trials). This thorough analysis monitoring the uncertainties in different steps of the data post processing (starting time of the background fitting, period of background fitting, background density, white noise) allowed for validation of the presented distance distributions.

### Results

Lysine and tyrosine derivatives comprising a 2,2,5,5-tetramethylpyrroline-1-oxyl-moiety were used in the following experiments as representative ncAAs.

The inventors subjected a library of ∼10⁸ PylRS mutants with random positions around the pyrrolysine binding pocket to an *in vivo* selection process based on amber suppression-dependent positive and negative reporter genes This identified three clones expressing the PylRS mutants (PylRS-SL1-3), which are capable of processing lysine derivatives and four clones expressing PylRS mutants (PylRS-SL4-7), which are capable of processing tyrosine derivatives (Figure 5). The identified PylRS mutants were found to comprise the following amino acid substitutions in comparison to wild type PylRS.

**Table 2: Summary of the amino acid substitutions of the PylRS mutants of the invention (substituted amino acids are indicated in bold letters)**

| **amino acid** | **302** | **306** | **309** | **346** | **348** | **384** | **413** | **415** |
|---|---|---|---|---|---|---|---|---|
| PylRS_wt | A | Y | L | N | C | Y | **I** | K |
| PylRS_AF | A | **A** | L | N | C | **F** | **I** | K |
| PylRS-SL1 | A | **A** | L | N | C | **F** | **L** | K |
| PylRS-SL2 | A | **A** | L | N | C | **F** | **L** | **R** |
| PylRS-SL3 | A | **A** | L | N | **T** | **F** | **L** | K |
| PylRS-SL4 | A | **A** | L | **T** | **W** | **F** | **I** | K |
| PylRS-SL5 | A | **A** | L | **V** | **K** | **F** | **I** | K |
| PylRS-SL6 | F | **A** | V | **A** | **Y** | **F** | **I** | K |
| PylRS-SL7 | A | **A** | L | **K** | C | **F** | **V** | K |

Clones co-expressing tRNA^{Pyl}/PylRS-SL1-3 and an in-frame amber mutant of chloramphenicol(Cam)-acetyltransferase (CAT_Q98TAG), exhibited growth on Cam media, both in presence of **2b** and **2a**, but did not grow in their absence, with PylRS-SL1 exhibiting the strongest growth. Likewise, Clones co-expressing tRNA^{Pyl}/PylRS-SL4-7 and an in-frame amber mutant of chloramphenicol(Cam)-acetyltransferase (CAT_Q98TAG), exhibited growth on Cam media, in presence of **3a** and **3b**, but did not grow in its absence (Figure 5) This suggests that the PylRS mutants indeed exhibit the envisaged cross-reactivity for lysine and tyrosine derivatives comprising a 2,2,5,5-tetramethylpyrrofine-1-oxyl-moiety, respectively, and promote their incorporation into proteins with high efficiency and fidelity.

For quantitative analysis, the inventors co-expressed tRNA^{Pyl}/PylRS-SL with an amber mutant of green fluorescent protein with C-terminal His6 tag (GFP-Y39TAG) in presence and absence of **2a** or **2b**. Cellular fluorescence as well as SDS-PAGE analysis of protein (purified by Ni-NTA chromatography that addresses the C-terminal His6 tag) revealed expression of full-length protein only in presence of **2a** or **2b** with similar levels (4.6 and 4.3 mg/L, Figure 1 C). Moreover, electrospray ionization tandem mass spectrometry (ESI MS/MS) of DTT-reduced, trypsin-digested GFP-Y39→**2a** revealed the expected presence of the reduced form **2b** at position 39 (Figure 1D). Finally, an equivalent expression employing **2a** and an amber mutant of thioredoxin (TRX-R74TAG) revealed expression only in presence of **2a** (Figure 1 E, three additional TRX amber mutants showed comparable results). These data indicate high efficiency and fidelity of incorporation in response to the amber codon.

To get insights into the *in vivo* stability of 2a, in particular in *E.coli*, and to generally test, if sufficient amounts of protein with sufficiency high labeling degrees (i.e. the percentage of intact, paramagnetic spin label present in a protein) for EPR distance measurements could be obtained, the inventors expressed GFP-Y39→**2a** in *E. coli* Top10 in presence of **2a** with time-resolved monitoring by both SDS PAGE analysis and EPR measurements. In view of potential DEER measurements, the inventors also tested the release factor 1 (RF1) knockout strain JX33 that previously enabled the incorporation of multiple non-canonical amino acids into one protein (Johnson et al., 2012). Expression levels were increasing up to an induction time of 6 h, with higher levels for JX33 (Figure 2A). Likewise, the purified proteins featured a nitroxide-characteristic EPR signal (for a full characterization of the EPR spectrum of **2a** (Figure 7) emerging at an induction time of 3 h and reaching a maximum at 6 h (Figure 2B).

Quantification of the labeling degree by EPR measurements revealed a fraction of 49 % intact spin label even after 8 h induction time in JX33 in presence of 1 mM **2a** (Figure 2C). When 3 mM **2a** was employed in the expression, a degree of 68 % was obtained, which provides a simple means to obtain increased labeling degrees. Under these conditions, a labeling degree of 52 % was obtained even after an induction time of 20 h. This shows that the 2,2,5,5-tetramethyl-pyrroline-1-oxyl spin label exhibits an exceptionally high stability in *E. coli* that strongly exceeds the stabilities that have previously been reported for eukaryotic environments. The induction time of 20 h is thereby at the upper end typically used for protein expressions in *E. coli.* This shows that the present approach affords high yields of proteins with sufficient labeling degrees for EPR measurements from *E. coli* expression cultures.

The genetic encoding of **2a** provides new perspectives to study proteins by in-cell EPR spectroscopy directly in the natural host environment where they are biosynthesized and processed. However, this requires sufficient sensitivity and the possibility to selectively observe the signal of **2a** that is incorporated into the target protein. In principle, intracellular **2a** that is present as free amino acid, as AMP-ester, esterified to tRNA^{Pyl} and potentially incorporated into amber-terminated host proteins could interfere. As a first proof of principle, the inventors performed expressions of *E. coli* TRX-R74→**2a** as above, washed the cells and directly subjected the cell pellets to EPR measurements. To assess potential background caused by the different forms of **2a**, the inventors included controls for all three orthogonal components required for genetic encoding, i.e. for **2a**, the tRNA^{Pyl/}PylRS-SL pair and the amber codon at position 74. Strikingly, only if all components were present, a significantly increased EPR signal could be detected (Figure 3A). This allowed recording the first EPR spectrum of an endogenous, spin-labeled protein in its natural host (Figure 3B).

To evaluate the potential for **2a** with respect to EPR distance measurements, the inventors expressed the doubly labeled protein mutant TRX-D141R74→**2a** in *E. coli* JX33 (Figure 4A). The protein was purified as above and dialyzed into aqueous buffer. Upon addition of 20 % (v/v) glycerol, the protein solution was shock-frozen in order to trap the macromolecular conformation. The distance measurements were performed at a temperature of 50 K. TRX-D14→**2a** was used as singly labeled control. The DEER data upon background correction is shown in Figure 4B. The DEER data of TRX-D14→**2a** is in full agreement with a homogeneous 3D distribution of spin labels as expected for singly labeled proteins in solution. The modulation depth of the TRX-D14/R74→**2a** DEER curve suggests that for approximately 6 % of the EPR-active TRX molecules both spin labels are intact, corresponding to an average labeling degree of 11 % per site. This indicates that DEER measurements are possible even with protein samples that exhibit lower per-site labeling degrees than observed in previous experiments.

The corresponding distance distribution was obtained by a model-free analysis using DEERAnalysis2013 (Figure 4). A systematic analysis of the influence of all steps in the data post-processing process was performed and resulted in the uncertainty intervals given in Figure 4D. In general, the width of the distance distribution reflects both the flexibility of TRX as well as the reorientational degree of freedom of **2a**, while the errors of EPR distance measurements are significantly smaller. The influence of the linker flexibility of the spin labels can be predicted via a rotamer approach. The experimental distance distribution for TRX-D14/R74→**2a** is in full agreement with the theoretically predicted distance distribution between conventional MTSSL spin labels and match distances expected from previous crystal structures (Figure 4A). Hence, it reveals **2a** as a useful structural probe with similar characteristics as MTSSL.

In conclusion, the inventors demonstrate the direct, genetically encoded biosynthesis of spin-labelled proteins in living cells. This completely eliminates the need for chemical spin labeling and significantly facilitates protein EPR studies. **2a** is remarkably stable in *E. coli* and can be incorporated at multiple sites within one protein for intramolecular EPR distance measurements. Furthermore, the employed tRNA^{Pyl}/pylRS pair exhibits orthogonality in all domains of life. Indeed, PylRS-SL1 supports amber suppression in presence of **2a** and **2b** in mammalian cell lines with high efficiency and fidelity (Figure 8). Consequently, general perspectives for facile in-cell EPR measurements of endogenous proteins directly in their natural host environment are provided.

### References

C. Altenbach, T. Marti, H. G. Khorana, W. L. Hubbell, Science 1990, 248, 1088.
Y. W. Chiang, P. P. Borbat, J. H. Freed, Journal of Magnetic Resonance 2005, 172, 279-295.
G. Jeschke, Ann. Rev. Phys. Chem. 2012, 63, 419;
bW. L. Hubbell, D. S. Cafiso, C. Altenbach, Nat. Struc. Biol. 2000, 7, 735
G. Jeschke, V. Chechik, P. lonita, A. Godt, H. Zimmermann, J. Banham, C. R. Timmel, D. Hilger, H. Jung, Appl Magn Reson 2006, 30, 473-498
G. Jeschke, M. Pannier, H. W. Spiess, Biological Magnetic Resonance, Vol. 12, Kluver Academic, New York, 2000.
D. B. F. Johnson, C. Wang, J. F. Xu, M. D. Schultz, R. J. Schmitz, J. R. Ecker, L. Wang, ACS Chem. Biol. 2012, 7, 1337
J. P. Klare, H. J. Steinhoff, Photosynth. Res. 2009, 102, 377
CC. Liu and PG. Schultz; Annu Rev Biochem. 2010;79:413-44.
H. S. McHaourab, M. A. Lietzow, K. Hideg, W. L. Hubbell, Biochemistry 1996, 35, 7692-7704.
T. Plass, S. Milles, C. Koehler, J. Szymanski, R. Mueller, M. Wiessler, C. Schultz, E. A. Lemke, Angew Chem Int Ed Engl 2012, 51, 4166-4170
A. Schweiger and G. Jeschke, Principles of pulse electron paramagnetic resonance, Oxford University Press, Oxford, 2005.
T. Yanagisawa, R. Ishii, R. Fukunaga, T. Kobayashi, K. Sakamoto, S. Yokoyama; Chem. Biol. 2008, 15,1187;

## Claims

1. A non-canonical amino acid of the formula I
A-L-X
wherein A is a lysine or a tyrosine,
L is a linker or absent, and
X is an aminoxyl radical,
wherein if A is lysine, L is bound to the N-epsilon atom of the lysine or, if L is absent, X is bound to the N-epsilon atom of the lysine;
and if A is tyrosine, L is bound to the phenolic hydroxyl of the tyrosine or, if L is absent, X is bound to the phenolic hydroxyl of the tyrosine.

2. The non-canonical amino acid of claim 1, wherein L is selected from the group consisting of an ether residue, a carbamate residue and an urea residue,

3. The non-canonical amino acid of claim 1 or 2, wherein the aminoxyl radical comprises an optionally substituted heterocycle, preferably an optionally substituted pyrroline residue, an optionally substituted oxazolidine residue or an optionally substituted piperidine residue.

4. The non-canonical amino acid of any of claims 1 to 3, wherein the aminoxyl radical comprises a 2,2,5,5-tetramethylpyrroline-1-oxyl residue, a 2,2,5,5-tetraethylpyrroline-1-oxyl residue, a 2,2,6,6-tetramethylpiperidine-1-oxyl residue, a 2,2,6,6-tetraethylpiperidine-1-oxyl residue, a 2,2,5,5-tetramethylpyrrofidine-1-oxyl residue, a 2,2,5,5-tetraethylpyrrolidine-1-oxyl residue or a 4,4-dimethyl-oxazolidine-*N*- oxyl residue.

5. A non-canonical amino acid selected from the group consisting of 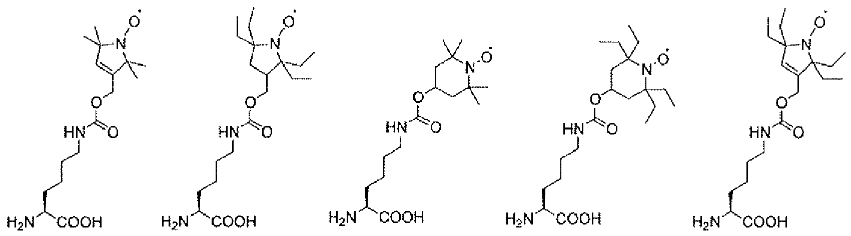 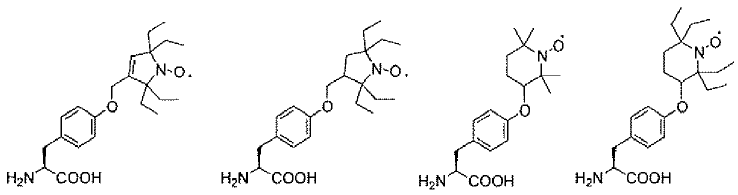 and

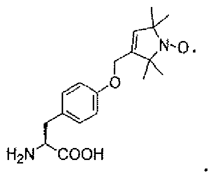

6. A protein comprising at least one, preferably two non-canonical amino acid according to any of claims 1 to 5.

7. A method for introducing a spin label into a protein comprising the steps of
- providing a nucleic acid sequence encoding for the protein,
- introducing a stop codon or a quadruplet codon into the sequence, and
- expressing the sequence in the presence of a tRNA^{Pyl}, a modified PylRS, and a non-canonical amino acid according to any of claims 1 to 5, which is encoded by the stop codon or the quadruplet codon.

8. A method for analyzing a protein comprising the steps of producing a derivative of the protein comprising at least one non-canonical amino acid according to any of claims 1 to 5 at a defined position, and recording the electron paramagnetic resonance spectrum of the derivative.

9. The method of claim 8, wherein the derivative comprises two non-canonical amino acids according to any of claims 1 to 5 at two distinct positions.

10. The method of claim 8, wherein the method further comprises the step of producing a derivative of a second protein comprising an non-canonical amino acid according to any of claims 1 to 5 at a defined position.

11. A modified pyrrolysyl-tRNA-synthetase (PylRS) having a substitution of alanine for tyrosine at position 306, a substitution of phenylalanine for tyrosine at position 384 and a substitution of leucine for isoleucine at position 413.

12. The modified PylRS of claim 11, further having a substitution of threonine for cysteine at position 348 and/or a substitution of arginine for lysine at position 415.

13. A modified pyrrolysyl-tRNA-synthetase (PylRS) having a substitution of alanine for tyrosine at position 306, a substitution of phenylalanine for tyrosine at position 384 and a substitution of threonine, alanine, valine or lysine for asparagine at position 346.

14. The modified PylRS of claim 13, further having at least one additional mutation at position 348 and/or 413.

15. Use of a non-canonical amino acid according to any of claims 1 to 5 for site-directed spin labeling.


SEQUENCE LISTING

<110>  Universität Konstanz

<120>  Genetically Encoded Spin Label

<130>  T30054

<160>  15

<170>  PatentIn version 3.3

<210>  1
<211>  454
<212>  PRT
<213>  Methanosarcina mazei

<400>  1

Met Asp Lys Lys Pro Leu Asn Thr Leu Ile Ser Ala Thr Gly Leu Trp
1               5                   10                  15

Met Ser Arg Thr Gly Thr Ile His Lys Ile Lys His His Glu Val Ser
            20                  25                  30

Arg Ser Lys Ile Tyr Ile Glu Met Ala Cys Gly Asp His Leu Val Val
        35                  40                  45

Asn Asn Ser Arg Ser Ser Arg Thr Ala Arg Ala Leu Arg His His Lys
    50                  55                  60

Tyr Arg Lys Thr Cys Lys Arg Cys Arg Val Ser Asp Glu Asp Leu Asn
65                  70                  75                  80

Lys Phe Leu Thr Lys Ala Asn Glu Asp Gln Thr Ser Val Lys Val Lys
                85                  90                  95

Val Val Ser Ala Pro Thr Arg Thr Lys Lys Ala Met Pro Lys Ser Val
            100                 105                 110

Ala Arg Ala Pro Lys Pro Leu Glu Asn Thr Glu Ala Ala Gln Ala Gln
        115                 120                 125

Pro Ser Gly Ser Lys Phe Ser Pro Ala Ile Pro Val Ser Thr Gln Glu
    130                 135                 140

Ser Val Ser Val Pro Ala Ser Val Ser Thr Ser Ile Ser Ser Ile Ser
145                 150                 155                 160

Thr Gly Ala Thr Ala Ser Ala Leu Val Lys Gly Asn Thr Asn Pro Ile
                165                 170                 175

Thr Ser Met Ser Ala Pro Val Gln Ala Ser Ala Pro Ala Leu Thr Lys
        180                 185                 190

Ser Gln Thr Asp Arg Leu Glu Val Leu Leu Asn Pro Lys Asp Glu Ile
    195                 200                 205

Ser Leu Asn Ser Gly Lys Pro Phe Arg Glu Leu Glu Ser Glu Leu Leu
210                 215                 220

Ser Arg Arg Lys Lys Asp Leu Gln Gln Ile Tyr Ala Glu Glu Arg Glu
225                 230                 235                 240

Asn Tyr Leu Gly Lys Leu Glu Arg Glu Ile Thr Arg Phe Phe Val Asp
                245                 250                 255

Arg Gly Phe Leu Glu Ile Lys Ser Pro Ile Leu Ile Pro Leu Glu Tyr
        260                 265                 270

Ile Glu Arg Met Gly Ile Asp Asn Asp Thr Glu Leu Ser Lys Gln Ile
    275                 280                 285

Phe Arg Val Asp Lys Asn Phe Cys Leu Arg Pro Met Leu Ala Pro Asn
290                 295                 300

Leu Tyr Asn Tyr Leu Arg Lys Leu Asp Arg Ala Leu Pro Asp Pro Ile
305                 310                 315                 320

Lys Ile Phe Glu Ile Gly Pro Cys Tyr Arg Lys Glu Ser Asp Gly Lys
                325                 330                 335

Glu His Leu Glu Glu Phe Thr Met Leu Asn Phe Cys Gln Met Gly Ser
        340                 345                 350

Gly Cys Thr Arg Glu Asn Leu Glu Ser Ile Ile Thr Asp Phe Leu Asn
    355                 360                 365

His Leu Gly Ile Asp Phe Lys Ile Val Gly Asp Ser Cys Met Val Tyr
370                 375                 380

Gly Asp Thr Leu Asp Val Met His Gly Asp Leu Glu Leu Ser Ser Ala
385                 390                 395                 400

Val Val Gly Pro Ile Pro Leu Asp Arg Glu Trp Gly Ile Asp Lys Pro
                405                 410                 415

Trp Ile Gly Ala Gly Phe Gly Leu Glu Arg Leu Leu Lys Val Lys His
        420                 425                 430

Asp Phe Lys Asn Ile Lys Arg Ala Ala Arg Ser Glu Ser Tyr Tyr Asn
        435                 440                 445

Gly Ile Ser Thr Asn Leu
    450

<210>  2
<211>  454
<212>  PRT
<213>  artificial

<220>
<223>  PylRS mutant

<400>  2

Met Asp Lys Lys Pro Leu Asn Thr Leu Ile Ser Ala Thr Gly Leu Trp
1               5                   10                  15

Met Ser Arg Thr Gly Thr Ile His Lys Ile Lys His His Glu Val Ser
            20                  25                  30

Arg Ser Lys Ile Tyr Ile Glu Met Ala Cys Gly Asp His Leu Val Val
        35                  40                  45

Asn Asn Ser Arg Ser Ser Arg Thr Ala Arg Ala Leu Arg His His Lys
    50                  55                  60

Tyr Arg Lys Thr Cys Lys Arg Cys Arg Val Ser Asp Glu Asp Leu Asn
65                  70                  75                  80

Lys Phe Leu Thr Lys Ala Asn Glu Asp Gln Thr Ser Val Lys Val Lys
                85                  90                  95

Val Val Ser Ala Pro Thr Arg Thr Lys Lys Ala Met Pro Lys Ser Val
            100                 105                 110

Ala Arg Ala Pro Lys Pro Leu Glu Asn Thr Glu Ala Ala Gln Ala Gln
        115                 120                 125

Pro Ser Gly Ser Lys Phe Ser Pro Ala Ile Pro Val Ser Thr Gln Glu
    130                 135                 140

Ser Val Ser Val Pro Ala Ser Val Ser Thr Ser Ile Ser Ser Ile Ser
145                 150                 155                 160

Thr Gly Ala Thr Ala Ser Ala Leu Val Lys Gly Asn Thr Asn Pro Ile
                165                 170                 175

Thr Ser Met Ser Ala Pro Val Gln Ala Ser Ala Pro Ala Leu Thr Lys
            180                 185                 190

Ser Gln Thr Asp Arg Leu Glu Val Leu Leu Asn Pro Lys Asp Glu Ile
        195                 200                 205

Ser Leu Asn Ser Gly Lys Pro Phe Arg Glu Leu Glu Ser Glu Leu Leu
    210                 215                 220

Ser Arg Arg Lys Lys Asp Leu Gln Gln Ile Tyr Ala Glu Glu Arg Glu
225                 230                 235                 240

Asn Tyr Leu Gly Lys Leu Glu Arg Glu Ile Thr Arg Phe Phe Val Asp
                245                 250                 255

Arg Gly Phe Leu Glu Ile Lys Ser Pro Ile Leu Ile Pro Leu Glu Tyr
            260                 265                 270

Ile Glu Arg Met Gly Ile Asp Asn Asp Thr Glu Leu Ser Lys Gln Ile
        275                 280                 285

Phe Arg Val Asp Lys Asn Phe Cys Leu Arg Pro Met Leu Ala Pro Asn
    290                 295                 300

Leu Ala Asn Tyr Leu Arg Lys Leu Asp Arg Ala Leu Pro Asp Pro Ile
305                 310                 315                 320

Lys Ile Phe Glu Ile Gly Pro Cys Tyr Arg Lys Glu Ser Asp Gly Lys
                325                 330                 335

Glu His Leu Glu Glu Phe Thr Met Leu Asn Phe Cys Gln Met Gly Ser
            340                 345                 350

Gly Cys Thr Arg Glu Asn Leu Glu Ser Ile Ile Thr Asp Phe Leu Asn
        355                 360                 365

His Leu Gly Ile Asp Phe Lys Ile Val Gly Asp Ser Cys Met Val Phe
    370                 375                 380

Gly Asp Thr Leu Asp Val Met His Gly Asp Leu Glu Leu Ser Ser Ala
385                 390                 395                 400

Val Val Gly Pro Ile Pro Leu Asp Arg Glu Trp Gly Leu Asp Lys Pro
                405                 410                 415

Trp Ile Gly Ala Gly Phe Gly Leu Glu Arg Leu Leu Lys Val Lys His
            420                 425                 430

Asp Phe Lys Asn Ile Lys Arg Ala Ala Arg Ser Glu Ser Tyr Tyr Asn
        435                 440                 445

Gly Ile Ser Thr Asn Leu
    450

<210>  3
<211>  454
<212>  PRT
<213>  artificial

<220>
<223>  PylRS mutant

<400>  3

Met Asp Lys Lys Pro Leu Asn Thr Leu Ile Ser Ala Thr Gly Leu Trp
1               5                   10                  15

Met Ser Arg Thr Gly Thr Ile His Lys Ile Lys His His Glu Val Ser
            20                  25                  30

Arg Ser Lys Ile Tyr Ile Glu Met Ala Cys Gly Asp His Leu Val Val
        35                  40                  45

Asn Asn Ser Arg Ser Ser Arg Thr Ala Arg Ala Leu Arg His His Lys
    50                  55                  60

Tyr Arg Lys Thr Cys Lys Arg Cys Arg Val Ser Asp Glu Asp Leu Asn
65                  70                  75                  80

Lys Phe Leu Thr Lys Ala Asn Glu Asp Gln Thr Ser Val Lys Val Lys
                85                  90                  95

Val Val Ser Ala Pro Thr Arg Thr Lys Lys Ala Met Pro Lys Ser Val
            100                 105                 110

Ala Arg Ala Pro Lys Pro Leu Glu Asn Thr Glu Ala Ala Gln Ala Gln
        115                 120                 125

Pro Ser Gly Ser Lys Phe Ser Pro Ala Ile Pro Val Ser Thr Gln Glu
    130                 135                 140

Ser Val Ser Val Pro Ala Ser Val Ser Thr Ser Ile Ser Ser Ile Ser
145                 150                 155                 160

Thr Gly Ala Thr Ala Ser Ala Leu Val Lys Gly Asn Thr Asn Pro Ile
                165                 170                 175

Thr Ser Met Ser Ala Pro Val Gln Ala Ser Ala Pro Ala Leu Thr Lys
        180                 185                 190

Ser Gln Thr Asp Arg Leu Glu Val Leu Leu Asn Pro Lys Asp Glu Ile
    195                 200                 205

Ser Leu Asn Ser Gly Lys Pro Phe Arg Glu Leu Glu Ser Glu Leu Leu
    210                 215                 220

Ser Arg Arg Lys Lys Asp Leu Gln Gln Ile Tyr Ala Glu Glu Arg Glu
225                 230                 235                 240

Asn Tyr Leu Gly Lys Leu Glu Arg Glu Ile Thr Arg Phe Phe Val Asp
                245                 250                 255

Arg Gly Phe Leu Glu Ile Lys Ser Pro Ile Leu Ile Pro Leu Glu Tyr
            260                 265                 270

Ile Glu Arg Met Gly Ile Asp Asn Asp Thr Glu Leu Ser Lys Gln Ile
        275                 280                 285

Phe Arg Val Asp Lys Asn Phe Cys Leu Arg Pro Met Leu Ala Pro Asn
    290                 295                 300

Leu Ala Asn Tyr Leu Arg Lys Leu Asp Arg Ala Leu Pro Asp Pro Ile
305                 310                 315                 320

Lys Ile Phe Glu Ile Gly Pro Cys Tyr Arg Lys Glu Ser Asp Gly Lys
                325                 330                 335

Glu His Leu Glu Glu Phe Thr Met Leu Asn Phe Cys Gln Met Gly Ser
            340                 345                 350

Gly Cys Thr Arg Glu Asn Leu Glu Ser Ile Ile Thr Asp Phe Leu Asn
        355                 360                 365

His Leu Gly Ile Asp Phe Lys Ile Val Gly Asp Ser Cys Met Val Phe
    370                 375                 380

Gly Asp Thr Leu Asp Val Met His Gly Asp Leu Glu Leu Ser Ser Ala
385                 390                 395                 400

Val Val Gly Pro Ile Pro Leu Asp Arg Glu Trp Gly Leu Asp Arg Pro
                405                 410                 415

Trp Ile Gly Ala Gly Phe Gly Leu Glu Arg Leu Leu Lys Val Lys His
            420                 425                 430

Asp Phe Lys Asn Ile Lys Arg Ala Ala Arg Ser Glu Ser Tyr Tyr Asn
        435                 440                 445

Gly Ile Ser Thr Asn Leu
    450

<210>  4
<211>  454
<212>  PRT
<213>  artificial

<220>
<223>  PylRS mutant

<400>  4

Met Asp Lys Lys Pro Leu Asn Thr Leu Ile Ser Ala Thr Gly Leu Trp
1               5                   10                  15

Met Ser Arg Thr Gly Thr Ile His Lys Ile Lys His His Glu Val Ser
            20                  25                  30

Arg Ser Lys Ile Tyr Ile Glu Met Ala Cys Gly Asp His Leu Val Val
        35                  40                  45

Asn Asn Ser Arg Ser Ser Arg Thr Ala Arg Ala Leu Arg His His Lys
    50                  55                  60

Tyr Arg Lys Thr Cys Lys Arg Cys Arg Val Ser Asp Glu Asp Leu Asn
65                  70                  75                  80

Lys Phe Leu Thr Lys Ala Asn Glu Asp Gln Thr Ser Val Lys Val Lys
                85                  90                  95

Val Val Ser Ala Pro Thr Arg Thr Lys Lys Ala Met Pro Lys Ser Val
            100                 105                 110

Ala Arg Ala Pro Lys Pro Leu Glu Asn Thr Glu Ala Ala Gln Ala Gln
        115                 120                 125

Pro Ser Gly Ser Lys Phe Ser Pro Ala Ile Pro Val Ser Thr Gln Glu
    130                 135                 140

Ser Val Ser Val Pro Ala Ser Val Ser Thr Ser Ile Ser Ser Ile Ser
145                 150                 155                 160

Thr Gly Ala Thr Ala Ser Ala Leu Val Lys Gly Asn Thr Asn Pro Ile
                165                 170                 175

Thr Ser Met Ser Ala Pro Val Gln Ala Ser Ala Pro Ala Leu Thr Lys
            180                 185                 190

Ser Gln Thr Asp Arg Leu Glu Val Leu Leu Asn Pro Lys Asp Glu Ile
        195                 200                 205

Ser Leu Asn Ser Gly Lys Pro Phe Arg Glu Leu Glu Ser Glu Leu Leu
    210                 215                 220

Ser Arg Arg Lys Lys Asp Leu Gln Gln Ile Tyr Ala Glu Glu Arg Glu
225                 230                 235                 240

Asn Tyr Leu Gly Lys Leu Glu Arg Glu Ile Thr Arg Phe Phe Val Asp
                245                 250                 255

Arg Gly Phe Leu Glu Ile Lys Ser Pro Ile Leu Ile Pro Leu Glu Tyr
            260                 265                 270

Ile Glu Arg Met Gly Ile Asp Asn Asp Thr Glu Leu Ser Lys Gln Ile
        275                 280                 285

Phe Arg Val Asp Lys Asn Phe Cys Leu Arg Pro Met Leu Ala Pro Asn
    290                 295                 300

Leu Ala Asn Tyr Leu Arg Lys Leu Asp Arg Ala Leu Pro Asp Pro Ile
305                 310                 315                 320

Lys Ile Phe Glu Ile Gly Pro Cys Tyr Arg Lys Glu Ser Asp Gly Lys
                325                 330                 335

Glu His Leu Glu Glu Phe Thr Met Leu Asn Phe Thr Gln Met Gly Ser
            340                 345                 350

Gly Cys Thr Arg Glu Asn Leu Glu Ser Ile Ile Thr Asp Phe Leu Asn
        355                 360                 365

His Leu Gly Ile Asp Phe Lys Ile Val Gly Asp Ser Cys Met Val Phe
    370                 375                 380

Gly Asp Thr Leu Asp Val Met His Gly Asp Leu Glu Leu Ser Ser Ala
385                 390                 395                 400

Val Val Gly Pro Ile Pro Leu Asp Arg Glu Trp Gly Leu Asp Lys Pro
                405                 410                 415

Trp Ile Gly Ala Gly Phe Gly Leu Glu Arg Leu Leu Lys Val Lys His
            420                 425                 430

Asp Phe Lys Asn Ile Lys Arg Ala Ala Arg Ser Glu Ser Tyr Tyr Asn
        435                 440                 445

Gly Ile Ser Thr Asn Leu
    450

<210>  5
<211>  454
<212>  PRT
<213>  artificial

<220>
<223>  PylRS mutant

<400>  5

Met Asp Lys Lys Pro Leu Asn Thr Leu Ile Ser Ala Thr Gly Leu Trp
1               5                   10                  15

Met Ser Arg Thr Gly Thr Ile His Lys Ile Lys His His Glu Val Ser
            20                  25                  30

Arg Ser Lys Ile Tyr Ile Glu Met Ala Cys Gly Asp His Leu Val Val
        35                  40                  45

Asn Asn Ser Arg Ser Ser Arg Thr Ala Arg Ala Leu Arg His His Lys
    50                  55                  60

Tyr Arg Lys Thr Cys Lys Arg Cys Arg Val Ser Asp Glu Asp Leu Asn
65                  70                  75                  80

Lys Phe Leu Thr Lys Ala Asn Glu Asp Gln Thr Ser Val Lys Val Lys
                85                  90                  95

Val Val Ser Ala Pro Thr Arg Thr Lys Lys Ala Met Pro Lys Ser Val
            100                 105                 110

Ala Arg Ala Pro Lys Pro Leu Glu Asn Thr Glu Ala Ala Gln Ala Gln
        115                 120                 125

Pro Ser Gly Ser Lys Phe Ser Pro Ala Ile Pro Val Ser Thr Gln Glu
    130                 135                 140

Ser Val Ser Val Pro Ala Ser Val Ser Thr Ser Ile Ser Ser Ile Ser
145                 150                 155                 160

Thr Gly Ala Thr Ala Ser Ala Leu Val Lys Gly Asn Thr Asn Pro Ile
                165                 170                 175

Thr Ser Met Ser Ala Pro Val Gln Ala Ser Ala Pro Ala Leu Thr Lys
        180                 185                 190

Ser Gln Thr Asp Arg Leu Glu Val Leu Leu Asn Pro Lys Asp Glu Ile
    195                 200                 205

Ser Leu Asn Ser Gly Lys Pro Phe Arg Glu Leu Glu Ser Glu Leu Leu
210                 215                 220

Ser Arg Arg Lys Lys Asp Leu Gln Gln Ile Tyr Ala Glu Glu Arg Glu
225                 230                 235                 240

Asn Tyr Leu Gly Lys Leu Glu Arg Glu Ile Thr Arg Phe Phe Val Asp
                245                 250                 255

Arg Gly Phe Leu Glu Ile Lys Ser Pro Ile Leu Ile Pro Leu Glu Tyr
            260                 265                 270

Ile Glu Arg Met Gly Ile Asp Asn Asp Thr Glu Leu Ser Lys Gln Ile
        275                 280                 285

Phe Arg Val Asp Lys Asn Phe Cys Leu Arg Pro Met Leu Ala Pro Asn
    290                 295                 300

Leu Ala Asn Tyr Leu Arg Lys Leu Asp Arg Ala Leu Pro Asp Pro Ile
305                 310                 315                 320

Lys Ile Phe Glu Ile Gly Pro Cys Tyr Arg Lys Glu Ser Asp Gly Lys
                325                 330                 335

Glu His Leu Glu Glu Phe Thr Met Leu Thr Phe Trp Gln Met Gly Ser
            340                 345                 350

Gly Cys Thr Arg Glu Asn Leu Glu Ser Ile Ile Thr Asp Phe Leu Asn
        355                 360                 365

His Leu Gly Ile Asp Phe Lys Ile Val Gly Asp Ser Cys Met Val Phe
    370                 375                 380

Gly Asp Thr Leu Asp Val Met His Gly Asp Leu Glu Leu Ser Ser Ala
385                 390                 395                 400

Val Val Gly Pro Ile Pro Leu Asp Arg Glu Trp Gly Ile Asp Lys Pro
                405                 410                 415

Trp Ile Gly Ala Gly Phe Gly Leu Glu Arg Leu Leu Lys Val Lys His
            420                 425                 430

Asp Phe Lys Asn Ile Lys Arg Ala Ala Arg Ser Glu Ser Tyr Tyr Asn
        435                 440                 445

Gly Ile Ser Thr Asn Leu
    450

<210>  6
<211>  454
<212>  PRT
<213>  artificial

<220>
<223>  PylRS mutant

<400>  6

Met Asp Lys Lys Pro Leu Asn Thr Leu Ile Ser Ala Thr Gly Leu Trp
1               5                   10                  15

Met Ser Arg Thr Gly Thr Ile His Lys Ile Lys His His Glu Val Ser
            20                  25                  30

Arg Ser Lys Ile Tyr Ile Glu Met Ala Cys Gly Asp His Leu Val Val
        35                  40                  45

Asn Asn Ser Arg Ser Ser Arg Thr Ala Arg Ala Leu Arg His His Lys
    50                  55                  60

Tyr Arg Lys Thr Cys Lys Arg Cys Arg Val Ser Asp Glu Asp Leu Asn
65                  70                  75                  80

Lys Phe Leu Thr Lys Ala Asn Glu Asp Gln Thr Ser Val Lys Val Lys
                85                  90                  95

Val Val Ser Ala Pro Thr Arg Thr Lys Lys Ala Met Pro Lys Ser Val
            100                 105                 110

Ala Arg Ala Pro Lys Pro Leu Glu Asn Thr Glu Ala Ala Gln Ala Gln
        115                 120                 125

Pro Ser Gly Ser Lys Phe Ser Pro Ala Ile Pro Val Ser Thr Gln Glu
    130                 135                 140

Ser Val Ser Val Pro Ala Ser Val Ser Thr Ser Ile Ser Ser Ile Ser
145                 150                 155                 160

Thr Gly Ala Thr Ala Ser Ala Leu Val Lys Gly Asn Thr Asn Pro Ile
                165                 170                 175

Thr Ser Met Ser Ala Pro Val Gln Ala Ser Ala Pro Ala Leu Thr Lys
        180                 185                 190

Ser Gln Thr Asp Arg Leu Glu Val Leu Leu Asn Pro Lys Asp Glu Ile
    195                 200                 205

Ser Leu Asn Ser Gly Lys Pro Phe Arg Glu Leu Glu Ser Glu Leu Leu
210                 215                 220

Ser Arg Arg Lys Lys Asp Leu Gln Gln Ile Tyr Ala Glu Glu Arg Glu
225                 230                 235                 240

Asn Tyr Leu Gly Lys Leu Glu Arg Glu Ile Thr Arg Phe Phe Val Asp
                245                 250                 255

Arg Gly Phe Leu Glu Ile Lys Ser Pro Ile Leu Ile Pro Leu Glu Tyr
            260                 265                 270

Ile Glu Arg Met Gly Ile Asp Asn Asp Thr Glu Leu Ser Lys Gln Ile
        275                 280                 285

Phe Arg Val Asp Lys Asn Phe Cys Leu Arg Pro Met Leu Ala Pro Asn
    290                 295                 300

Leu Ala Asn Tyr Leu Arg Lys Leu Asp Arg Ala Leu Pro Asp Pro Ile
305                 310                 315                 320

Lys Ile Phe Glu Ile Gly Pro Cys Tyr Arg Lys Glu Ser Asp Gly Lys
                325                 330                 335

Glu His Leu Glu Glu Phe Thr Met Leu Val Phe Lys Gln Met Gly Ser
            340                 345                 350

Gly Cys Thr Arg Glu Asn Leu Glu Ser Ile Ile Thr Asp Phe Leu Asn
        355                 360                 365

His Leu Gly Ile Asp Phe Lys Ile Val Gly Asp Ser Cys Met Val Phe
    370                 375                 380

Gly Asp Thr Leu Asp Val Met His Gly Asp Leu Glu Leu Ser Ser Ala
385                 390                 395                 400

Val Val Gly Pro Ile Pro Leu Asp Arg Glu Trp Gly Ile Asp Lys Pro
                405                 410                 415

Trp Ile Gly Ala Gly Phe Gly Leu Glu Arg Leu Leu Lys Val Lys His
            420                 425                 430

Asp Phe Lys Asn Ile Lys Arg Ala Ala Arg Ser Glu Ser Tyr Tyr Asn
        435                 440                 445

Gly Ile Ser Thr Asn Leu
    450

<210>  7
<211>  454
<212>  PRT
<213>  artificial

<220>
<223>  PylSR mutant

<400>  7

Met Asp Lys Lys Pro Leu Asn Thr Leu Ile Ser Ala Thr Gly Leu Trp
1               5                   10                  15

Met Ser Arg Thr Gly Thr Ile His Lys Ile Lys His His Glu Val Ser
            20                  25                  30

Arg Ser Lys Ile Tyr Ile Glu Met Ala Cys Gly Asp His Leu Val Val
        35                  40                  45

Asn Asn Ser Arg Ser Ser Arg Thr Ala Arg Ala Leu Arg His His Lys
    50                  55                  60

Tyr Arg Lys Thr Cys Lys Arg Cys Arg Val Ser Asp Glu Asp Leu Asn
65                  70                  75                  80

Lys Phe Leu Thr Lys Ala Asn Glu Asp Gln Thr Ser Val Lys Val Lys
                85                  90                  95

Val Val Ser Ala Pro Thr Arg Thr Lys Lys Ala Met Pro Lys Ser Val
            100                 105                 110

Ala Arg Ala Pro Lys Pro Leu Glu Asn Thr Glu Ala Ala Gln Ala Gln
        115                 120                 125

Pro Ser Gly Ser Lys Phe Ser Pro Ala Ile Pro Val Ser Thr Gln Glu
    130                 135                 140

Ser Val Ser Val Pro Ala Ser Val Ser Thr Ser Ile Ser Ser Ile Ser
145                 150                 155                 160

Thr Gly Ala Thr Ala Ser Ala Leu Val Lys Gly Asn Thr Asn Pro Ile
                165                 170                 175

Thr Ser Met Ser Ala Pro Val Gln Ala Ser Ala Pro Ala Leu Thr Lys
        180                 185                 190

Ser Gln Thr Asp Arg Leu Glu Val Leu Leu Asn Pro Lys Asp Glu Ile
    195                 200                 205

Ser Leu Asn Ser Gly Lys Pro Phe Arg Glu Leu Glu Ser Glu Leu Leu
    210                 215                 220

Ser Arg Arg Lys Lys Asp Leu Gln Gln Ile Tyr Ala Glu Glu Arg Glu
225                 230                 235                 240

Asn Tyr Leu Gly Lys Leu Glu Arg Glu Ile Thr Arg Phe Phe Val Asp
                245                 250                 255

Arg Gly Phe Leu Glu Ile Lys Ser Pro Ile Leu Ile Pro Leu Glu Tyr
        260                 265                 270

Ile Glu Arg Met Gly Ile Asp Asn Asp Thr Glu Leu Ser Lys Gln Ile
    275                 280                 285

Phe Arg Val Asp Lys Asn Phe Cys Leu Arg Pro Met Leu Phe Pro Asn
    290                 295                 300

Leu Ala Asn Tyr Val Arg Lys Leu Asp Arg Ala Leu Pro Asp Pro Ile
305                 310                 315                 320

Lys Ile Phe Glu Ile Gly Pro Cys Tyr Arg Lys Glu Ser Asp Gly Lys
                325                 330                 335

Glu His Leu Glu Glu Phe Thr Met Leu Ala Phe Tyr Gln Met Gly Ser
        340                 345                 350

Gly Cys Thr Arg Glu Asn Leu Glu Ser Ile Ile Thr Asp Phe Leu Asn
    355                 360                 365

His Leu Gly Ile Asp Phe Lys Ile Val Gly Asp Ser Cys Met Val Phe
    370                 375                 380

Gly Asp Thr Leu Asp Val Met His Gly Asp Leu Glu Leu Ser Ser Ala
385                 390                 395                 400

Val Val Gly Pro Ile Pro Leu Asp Arg Glu Trp Gly Ile Asp Lys Pro
                405                 410                 415

Trp Ile Gly Ala Gly Phe Gly Leu Glu Arg Leu Leu Lys Val Lys His
        420                 425                 430

Asp Phe Lys Asn Ile Lys Arg Ala Ala Arg Ser Glu Ser Tyr Tyr Asn
        435                 440                 445

Gly Ile Ser Thr Asn Leu
    450

<210>  8
<211>  454
<212>  PRT
<213>  artificial

<220>
<223>  PxlRS mutant

<400>  8

Met Asp Lys Lys Pro Leu Asn Thr Leu Ile Ser Ala Thr Gly Leu Trp
1               5                   10                  15

Met Ser Arg Thr Gly Thr Ile His Lys Ile Lys His His Glu Val Ser
            20                  25                  30

Arg Ser Lys Ile Tyr Ile Glu Met Ala Cys Gly Asp His Leu Val Val
        35                  40                  45

Asn Asn Ser Arg Ser Ser Arg Thr Ala Arg Ala Leu Arg His His Lys
    50                  55                  60

Tyr Arg Lys Thr Cys Lys Arg Cys Arg Val Ser Asp Glu Asp Leu Asn
65                  70                  75                  80

Lys Phe Leu Thr Lys Ala Asn Glu Asp Gln Thr Ser Val Lys Val Lys
                85                  90                  95

Val Val Ser Ala Pro Thr Arg Thr Lys Lys Ala Met Pro Lys Ser Val
            100                 105                 110

Ala Arg Ala Pro Lys Pro Leu Glu Asn Thr Glu Ala Ala Gln Ala Gln
        115                 120                 125

Pro Ser Gly Ser Lys Phe Ser Pro Ala Ile Pro Val Ser Thr Gln Glu
    130                 135                 140

Ser Val Ser Val Pro Ala Ser Val Ser Thr Ser Ile Ser Ser Ile Ser
145                 150                 155                 160

Thr Gly Ala Thr Ala Ser Ala Leu Val Lys Gly Asn Thr Asn Pro Ile
                165                 170                 175

Thr Ser Met Ser Ala Pro Val Gln Ala Ser Ala Pro Ala Leu Thr Lys
            180                 185                 190

Ser Gln Thr Asp Arg Leu Glu Val Leu Leu Asn Pro Lys Asp Glu Ile
        195                 200                 205

Ser Leu Asn Ser Gly Lys Pro Phe Arg Glu Leu Glu Ser Glu Leu Leu
    210                 215                 220

Ser Arg Arg Lys Lys Asp Leu Gln Gln Ile Tyr Ala Glu Glu Arg Glu
225                 230                 235                 240

Asn Tyr Leu Gly Lys Leu Glu Arg Glu Ile Thr Arg Phe Phe Val Asp
                245                 250                 255

Arg Gly Phe Leu Glu Ile Lys Ser Pro Ile Leu Ile Pro Leu Glu Tyr
            260                 265                 270

Ile Glu Arg Met Gly Ile Asp Asn Asp Thr Glu Leu Ser Lys Gln Ile
        275                 280                 285

Phe Arg Val Asp Lys Asn Phe Cys Leu Arg Pro Met Leu Ala Pro Asn
    290                 295                 300

Leu Ala Asn Tyr Leu Arg Lys Leu Asp Arg Ala Leu Pro Asp Pro Ile
305                 310                 315                 320

Lys Ile Phe Glu Ile Gly Pro Cys Tyr Arg Lys Glu Ser Asp Gly Lys
                325                 330                 335

Glu His Leu Glu Glu Phe Thr Met Leu Lys Phe Cys Gln Met Gly Ser
            340                 345                 350

Gly Cys Thr Arg Glu Asn Leu Glu Ser Ile Ile Thr Asp Phe Leu Asn
        355                 360                 365

His Leu Gly Ile Asp Phe Lys Ile Val Gly Asp Ser Cys Met Val Phe
    370                 375                 380

Gly Asp Thr Leu Asp Val Met His Gly Asp Leu Glu Leu Ser Ser Ala
385                 390                 395                 400

Val Val Gly Pro Ile Pro Leu Asp Arg Glu Trp Gly Val Asp Lys Pro
                405                 410                 415

Trp Ile Gly Ala Gly Phe Gly Leu Glu Arg Leu Leu Lys Val Lys His
            420                 425                 430

Asp Phe Lys Asn Ile Lys Arg Ala Ala Arg Ser Glu Ser Tyr Tyr Asn
        435                 440                 445

Gly Ile Ser Thr Asn Leu
    450

<210>  9
<211>  1365
<212>  DNA
<213>  artificial

<220>
<223>  PylRS mutant

<400>  9
   atggacaaaa aaccgctgaa taccctgatc tctgctactg gtctgtggat gagtcgtacc      60
   ggaaccattc ataaaatcaa acaccacgag gttagccgtt cgaaaatcta tattgagatg     120
   gcgtgtggcg atcatctggt tgtgaacaat agccgctctt ctcgtacagc acgtgcactg     180
   cgtcaccaca aatatcgtaa aacctgtaaa cgttgccgtg tgtccgatga ggatctgaac     240
   aaattcctga caaaagccaa tgaggaccaa acaagcgtga aagtgaaagt cgttagcgct     300
   cctacccgta ctaaaaaagc aatgccgaaa tccgttgctc gtgcccctaa accactggaa     360
   aacactgaag cagcacaggc acagccgtct ggaagcaaat tctctccggc cattcctgtt     420
   tctacccagg agtccgtttc tgttccagca agtgtgagca ccagcattag cagtattagc     480
   accggtgcca ccgctagcgc cctggttaaa ggcaatacca atccgattac aagcatgtct     540
   gccccggttc aagcatcagc tccagcactg acaaaatccc aaaccgatcg tctggaggtt     600
   ctgctgaatc cgaaagacga aatcagcctg aactccggca aaccgtttcg tgaactggag     660
   agcgaactgc tgtcacgtcg taaaaaagac ctgcaacaaa tctatgccga agaacgtgag     720
   aactatctgg ggaaactgga acgtgaaatc acccgctttt tcgtggatcg tggctttctg     780
   gagatcaaat ccccgattct gattcctctg gagtatatcg agcgtatggg catcgacaat     840
   gataccgaac tgagcaaaca aattttccgt gtggataaaa acttctgtct gcgccctatg     900
   ctagcaccaa atctggctaa ctatctgcgc aaactggacc gtgccctgcc tgatcctatc     960
   aaaatcttcg agatcggccc gtgttatcgt aaagagtccg acggtaaaga acatctggag    1020
   gagtttacca tgctgaactt ttgccaaatg ggttcaggtt gtactcgtga gaacctggaa    1080
   agcatcatca ccgattttct gaaccacctg ggcattgact tcaaaattgt gggcgacagc    1140
   tgtatggtgt ttggcgacac cctggatgtc atgcacggcg acctggaact gtctagtgcc    1200
   gttgttggac caattccgct ggaccgtgag tggggtttgg acaagccgtg gatcggagca    1260
   ggattcggtc tggaacgcct gctgaaagtg aaacacgact tcaaaaacat caaacgtgcc    1320
   gcccgttctg aatcgtatta taacggatct tctacgaacc tgtaa                     1365

<210>  10
<211>  1365
<212>  DNA
<213>  artificial

<220>
<223>  mutant PylRS

<400>  10
atggacaaaa aaccgctgaa taccctgatc tctgctactg gtctgtggat gagtcgtacc      60

ggaaccattc ataaaatcaa acaccacgag gttagccgtt cgaaaatcta tattgagatg     120

gcgtgtggcg atcatctggt tgtgaacaat agccgctctt ctcgtacagc acgtgcactg     180

cgtcaccaca aatatcgtaa aacctgtaaa cgttgccgtg tgtccgatga ggatctgaac     240

aaattcctga caaaagccaa tgaggaccaa acaagcgtga aagtgaaagt cgttagcgct     300

cctacccgta ctaaaaaagc aatgccgaaa tccgttgctc gtgcccctaa accactggaa     360

aacactgaag cagcacaggc acagccgtct ggaagcaaat tctctccggc cattcctgtt     420

tctacccagg agtccgtttc tgttccagca agtgtgagca ccagcattag cagtattagc     480

accggtgcca ccgctagcgc cctggttaaa ggcaatacca atccgattac aagcatgtct     540

gccccggttc aagcatcagc tccagcactg acaaaatccc aaaccgatcg tctggaggtt     600

ctgctgaatc cgaaagacga aatcagcctg aactccggca aaccgtttcg tgaactggag     660

agcgaactgc tgtcacgtcg taaaaaagac ctgcaacaaa tctatgccga agaacgtgag     720

aactatctgg ggaaactgga acgtgaaatc acccgctttt tcgtggatcg tggctttctg     780

gagatcaaat ccccgattct gattcctctg gagtatatcg agcgtatggg catcgacaat     840

gataccgaac tgagcaaaca aattttccgt gtggataaaa acttctgtct gcgccctatg     900

ctagcaccaa atctggctaa ctatctgcgc aaactggacc gtgccctgcc tgatcctatc     960

aaaatcttcg agatcggccc gtgttatcgt aaagagtccg acggtaaaga acatctggag    1020

gagtttacca tgctgaactt ttgccaaatg ggttcaggtt gtactcgtga gaacctggaa    1080

agcatcatca ccgattttct gaaccacctg ggcattgact tcaaaattgt gggcgacagc    1140

tgtatggtgt ttggcgacac cctggatgtc atgcacggcg acctggaact gtctagtgcc    1200

gttgttggac caattccgct ggaccgtgag tggggtctgg acaggccgtg gatcggagca    1260

ggattcggtc tggaacgcct gctgaaagtg aaacacgact tcaaaaacat caaacgtgcc    1320

gcccgttctg aatcgtatta taacgggatc tctacgaacc tgtaa                     1365

<210>  11
<211>  1365
<212>  DNA
<213>  artificial

<220>
<223>  mutant PylRS

<400>  11
atggacaaaa aaccgctgaa taccctgatc tctgctactg gtctgtggat gagtcgtacc      60

ggaaccattc ataaaatcaa acaccacgag gttagccgtt cgaaaatcta tattgagatg     120

gcgtgtggcg atcatctggt tgtgaacaat agccgctctt ctcgtacagc acgtgcactg     180

cgtcaccaca aatatcgtaa aacctgtaaa cgttgccgtg tgtccgatga ggatctgaac     240

aaattcctga caaaagccaa tgaggaccaa acaagcgtga aagtgaaagt cgttagcgct     300

cctacccgta ctaaaaaagc aatgccgaaa tccgttgctc gtgcccctaa accactggaa     360

aacactgaag cagcacaggc acagccgtct ggaagcaaat tctctccggc cattcctgtt     420

tctacccagg agtccgtttc tgttccagca agtgtgagca ccagcattag cagtattagc     480

accggtgcca ccgctagcgc cctggttaaa ggcaatacca atccgattac aagcatgtct     540

gccccggttc aagcatcagc tccagcactg acaaaatccc aaaccgatcg tctggaggtt     600

ctgctgaatc cgaaagacga aatcagcctg aactccggca aaccgtttcg tgaactggag     660

agcgaactgc tgtcacgtcg taaaaaagac ctgcaacaaa tctatgccga agaacgtgag     720

aactatctgg ggaaactgga acgtgaaatc acccgctttt tcgtggatcg tggctttctg     780

gagatcaaat ccccgattct gattcctctg gagtatatcg agcgtatggg catcgacaat     840

gataccgaac tgagcaaaca aattttccgt gtggataaaa acttctgtct gcgccctatg     900

ctagcaccaa atctggctaa ctatctgcgc aaactggacc gtgccctgcc tgatcctatc     960

aaaatcttcg agatcggccc gtgttatcgt aaagagtccg acggtaaaga acatctggag    1020

gagtttacca tgctgaactt tacgcaaatg ggttcaggtt gtactcgtga gaacctggaa    1080

agcatcatca ccgattttct gaaccacctg ggcattgact tcaaaattgt gggcgacagc    1140

tgtatggtgt ttggcgacac cctggatgtc atgcacggcg acctggaact gtctagtgcc    1200

gttgttggac caattccgct ggaccgtgag tggggtctgg acaagccgtg gatcggagca    1260

ggattcggtc tggaacgcct gctgaaagtg aaacacgact tcaaaaacat caaacgtgcc    1320

gcccgttctg aatcgtatta taacgggatc tctacgaacc tgtaa                    1365


<210>  12
<211>  1365
<212>  DNA
<213>  artificial

<220>
<223>  mutant PylRS

<400>  12
atggacaaaa aaccgctgaa taccctgatc tctgctactg gtctgtggat gagtcgtacc      60

ggaaccattc ataaaatcaa acaccacgag gttagccgtt cgaaaatcta tattgagatg     120

gcgtgtggcg atcatctggt tgtgaacaat agccgctctt ctcgtacagc acgtgcactg 180

cgtcaccaca aatatcgtaa aacctgtaaa cgttgccgtg tgtccgatga ggatctgaac 240

aaattcctga caaaagccaa tgaggaccaa acaagcgtga aagtgaaagt cgttagcgct 300

cctacccgta ctaaaaaagc aatgccgaaa tccgttgctc gtgcccctaa accactggaa 360

aacactgaag cagcacaggc acagccgtct ggaagcaaat tctctccggc cattcctgtt 420

tctacccagg agtccgtttc tgttccagca agtgtgagca ccagcattag cagtattagc 480

accggtgcca ccgctagcgc cctggttaaa ggcaatacca atccgattac aagcatgtct 540

gccccggttc aagcatcagc tccagcactg acaaaatccc aaaccgatcg tctggaggtt 600

ctgctgaatc cgaaagacga aatcagcctg aactccggca aaccgtttcg tgaactggag 660

agcgaactgc tgtcacgtcg taaaaaagac ctgcaacaaa tctatgccga agaacgtgag 720

aactatctgg ggaaactgga acgtgaaatc acccgctttt tcgtggatcg tggctttctg 780

gagatcaaat ccccgattct gattcctctg gagtatatcg agcgtatggg catcgacaat 840

gataccgaac tgagcaaaca aattttccgt gtggataaaa acttctgtct gcgccctatg 900

ctagcaccaa atctggctaa ctatctgcgc aaactggacc gtgccctgcc tgatcctatc 960

aaaatcttcg agatcggccc gtgttatcgt aaagagtccg acggtaaaga acatctggag 1020

gagtttacca tgctgacttt ttggcaaatg ggttcaggtt gtactcgtga gaacctggaa 1080

agcatcatca ccgattttct gaaccacctg ggcattgact tcaaaattgt gggcgacagc 1140

tgtatggtgt ttggcgacac cctggatgtc atgcacggcg acctggaact gtctagtgcc 1200

gttgttggac caattccgct ggaccgtgag tggggtatcg acaaaccgtg gatcggagca 1260

ggattcggtc tggaacgcct gctgaaagtg aaacacgact tcaaaaacat caaacgtgcc 1320

gcccgttctg aatcgtatta taacgggatc tctacgaacc tgtaa 1365

<210> 13
<211> 1365
<212> DNA
<213> artificial

<220>
<223> mutant PylRD

<400> 13
atggacaaaa aaccgctgaa taccctgatc tctgctactg gtctgtggat gagtcgtacc 60

ggaaccattc ataaaatcaa acaccacgag gttagccgtt cgaaaatcta tattgagatg 120

gcgtgtggcg atcatctggt tgtgaacaat agccgctctt ctcgtacagc acgtgcactg 180

cgtcaccaca aatatcgtaa aacctgtaaa cgttgccgtg tgtccgatga ggatctgaac 240

aaattcctga caaaagccaa tgaggaccaa acaagcgtga aagtgaaagt cgttagcgct 300

cctacccgta ctaaaaaagc aatgccgaaa tccgttgctc gtgcccctaa accactggaa 360

aacactgaag cagcacaggc acagccgtct ggaagcaaat tctctccggc cattcctgtt 420

tctacccagg agtccgtttc tgttccagca agtgtgagca ccagcattag cagtattagc 480

accggtgcca ccgctagcgc cctggttaaa ggcaatacca atccgattac aagcatgtct 540

gccccggttc aagcatcagc tccagcactg acaaaatccc aaaccgatcg tctggaggtt 600

ctgctgaatc cgaaagacga aatcagcctg aactccggca aaccgtttcg tgaactggag 660

agcgaactgc tgtcacgtcg taaaaaagac ctgcaacaaa tctatgccga agaacgtgag 720

aactatctgg ggaaactgga acgtgaaatc acccgctttt tcgtggatcg tggctttctg 780

gagatcaaat ccccgattct gattcctctg gagtatatcg agcgtatggg catcgacaat 840

gataccgaac tgagcaaaca aattttccgt gtggataaaa acttctgtct gcgccctatg 900

ctagcaccaa atctggctaa ctatctgcgc aaactggacc gtgccctgcc tgatcctatc 960

aaaatcttcg agatcggccc gtgttatcgt aaagagtccg acggtaaaga acatctggag 1020

gagtttacca tgctggtgtt taagcaaatg ggttcaggtt gtactcgtga gaacctggaa 1080

agcatcatca ccgattttct gaaccacctg ggcattgact tcaaaattgt gggcgacagc 1140

tgtatggtgt ttggcgacac cctggatgtc atgcacggcg acctggaact gtctagtgcc 1200

gttgttggac caattccgct ggaccgtgag tggggtatcg acaaaccgtg gatcggagca 1260

ggattcggtc tggaacgcct gctgaaagtg aaacacgact tcaaaaacat caaacgtgcc 1320

gcccgttctg aatcgtatta taacgggatc tctacgaacc tgtaa 1365

<210> 14
<211> 1365
<212> DNA
<213> artificial

<220>
<223> mutant PylRS

<400> 14
   atggacaaaa aaccgctgaa taccctgatc tctgctactg gtctgtggat gagtcgtacc 60
   ggaaccattc ataaaatcaa acaccacgag gttagccgtt cgaaaatcta tattgagatg 120
   gcgtgtggcg atcatctggt tgtgaacaat agccgctctt ctcgtacagc acgtgcactg 180
   cgtcaccaca aatatcgtaa aacctgtaaa cgttgccgtg tgtccgatga ggatctgaac 240
   aaattcctga caaaagccaa tgaggaccaa acaagcgtga aagtgaaagt cgttagcgct 300
   cctacccgta ctaaaaaagc aatgccgaaa tccgttgctc gtgcccctaa accactggaa 360
   aacactgaag cagcacaggc acagccgtct ggaagcaaat tctctccggc cattcctgtt 420
   tctacccagg agtccgtttc tgttccagca agtgtgagca ccagcattag cagtattagc 480
   accggtgcca ccgctagcgc cctggttaaa ggcaatacca atccgattac aagcatgtct 540
   gccccggttc aagcatcagc tccagcactg acaaaatccc aaaccgatcg tctggaggtt 600
   ctgctgaatc cgaaagacga aatcagcctg aactccggca aaccgtttcg tgaactggag 660
   agcgaactgc tgtcacgtcg taaaaaagac ctgcaacaaa tctatgccga agaacgtgag 720
   aactatctgg ggaaactgga acgtgaaatc acccgctttt tcgtggatcg tggctttctg 780
   gagatcaaat ccccgattct gattcctctg gagtatatcg agcgtatggg catcgacaat 840
   gataccgaac tgagcaaaca aattttccgt gtggataaaa acttctgtct gcgccctatg 900
   ctatttccaa atctggctaa ctatgtgcgc aaactggacc gtgccctgcc tgatcctatc 960
   aaaatcttcg agatcggccc gtgttatcgt aaagagtccg acggtaaaga acatctggag 1020
   gagtttacca tgctggcttt ttatcaaatg ggttcaggtt gtactcgtga gaacctggaa 1080
   agcatcatca ccgatttctt gaaccacctg ggcattgact tcaaaattgt gggcgacagc 1140
   tgtatggtgt ttggcgacac cctggatgtc atgcacggcg acctggaact gtctagtgcc 1200
   gttgttggac caattccgct ggaccgtgag tggggtatcg acaaaccgtg gatcggagca 1260
   ggattcggtc tggaacgcct gctgaaagtg aaacacgact tcaaaaacat caaacgtgcc 1320
   gcccgttctg aatcgtatta taacgggatc tctacgaacc tgtaa 1365

<210> 15
<211> 1365
<212> DNA
<213> artificial

<220>
<223> mutant PylRS

<400> 15
atggacaaaa aaccgctgaa taccctgatc tctgctactg gtctgtggat gagtcgtacc 60

ggaaccattc ataaaatcaa acaccacgag gttagccgtt cgaaaatcta tattgagatg 120

gcgtgtggcg atcatctggt tgtgaacaat agccgctctt ctcgtacagc acgtgcactg 180

cgtcaccaca aatatcgtaa aacctgtaaa cgttgccgtg tgtccgatga ggatctgaac 240

aaattcctga caaaagccaa tgaggaccaa acaagcgtga aagtgaaagt cgttagcgct 300

cctacccgta ctaaaaaagc aatgccgaaa tccgttgctc gtgcccctaa accactggaa 360

aacactgaag cagcacaggc acagccgtct ggaagcaaat tctctccggc cattcctgtt 420

tctacccagg agtccgtttc tgttccagca agtgtgagca ccagcattag cagtattagc 480

accggtgcca ccgctagcgc cctggttaaa ggcaatacca atccgattac aagcatgtct 540

gccccggttc aagcatcagc tccagcactg acaaaatccc aaaccgatcg tctggaggtt 600

ctgctgaatc cgaaagacga aatcagcctg aactccggca aaccgtttcg tgaactggag 660

agcgaactgc tgtcacgtcg taaaaaagac ctgcaacaaa tctatgccga agaacgtgag 720

aactatctgg ggaaactgga acgtgaaatc acccgctttt tcgtggatcg tggctttctg 780

gagatcaaat ccccgattct gattcctctg gagtatatcg agcgtatggg catcgacaat 840

gataccgaac tgagcaaaca aattttccgt gtggataaaa acttctgtct gcgccctatg 900

ctagcaccaa atctggctaa ctatctgcgc aaactggacc gtgccctgcc tgatcctatc 960

aaaatcttcg agatcggccc gtgttatcgt aaagagtccg acggtaaaga acatctggag 1020

gagtttacca tgctgaagtt ttgtcaaatg ggttcaggtt gtactcgtga gaacctggaa 1080

agcatcatca ccgattttct gaaccacctg ggcattgact tcaaaattgt gggcgacagc 1140

tgtatggtgt ttggcgacac cctggatgtc atgcacggcg acctggaact gtctagtgcc 1200

gttgttggac caattccgct ggaccgtgag tggggtgtgg acaaaccgtg gatcggagca 1260

ggattcggtc tggaacgcct gctgaaagtg aaacacgact tcaaaaacat caaacgtgcc 1320

gcccgttctg aatcgtatta taacgggatc tctacgaacc tgtaa 1365